# EUROPÄISCHE PATENTANMELDUNG

(11) **EP 1 132 471 A2**
(43) Veröffentlichungstag der Anmeldung: **12.09.2001**
(21) Anmeldenummer: 01108117.1
(22) Anmeldetag: 31.08.1990
(51) Int. Cl.: C12N 15/11, C07K 14/715, C07K 16/28

(54) **TNF-bindende Proteine**

(30) Priorität: 12.09.1989 CH 331989; 08.03.1990 CH 74690; 20.04.1990 CH 134790
(62) Teilanmeldung aus: 99100703.0
(71) Anmelder: F. HOFFMANN-LA ROCHE AG, 4070 Basel (CH)
(72) Erfinder: Brockhaus, Manfred, Dr., 4126 Bettingen (CH); Dembic, Zlatko, Dr., 4053 Basel (CH); Gentz, Reiner, Dr., 79618 Rheinfelden (DE); Lesslauer, Werner, Dr., 4059 Basel (CH); Lötscher, Hansruedi, Dr., 4313 Möhlin (CH); Schlaeger, Ernst-Jürgen, Dr., 79588 Efringen-Kirchen (DE)
(74) Vertreter: Keller, Günter, Dr.

(57) **Zusammenfassung**

Die vorliegende Erfindung betrifft nichtlösliche Proteine sowie lösliche oder nichtlösliche Fragmente davon, die TNF binden, in homogener Form, sowie deren physilogisch verträglichen Salze, insbesondere solche Proteine mit einem Molekulargewicht von etwa 55 oder 75 kD (nichtreduzierende SDS-PAGE-Bedingungen), Verfahren zur Isolierung solcher Proteine, Antikörper gegen solche Proteine, DNA-Sequenzen, die für nichtlösliche Proteine sowie lösliche oder nichtlösliche Fragmente davon, die TNF binden, kodieren, wie solche, die für Proteine kodieren, die zum einen Teil aus einem löslichen Fragment das TNF bindet und zum anderen Teil aus allen Domänen ausser der ersten der konstanten Region der schweren Kette humaner Immunglobuline bestehen und die davon kodierten rekombinanten Proteine wie Verfahren zur deren Herstellung mittels transformierter pro- wie eukaryotischer Wirtszellen.

## Beschreibung

Tumor Nekrosis Faktor α (TNFα, auch Cachectin), auf Grund seiner haemorragisch-nekrotisierenden Wirkung auf bestimmte Tumoren entdeckt, und Lymphotoxin (TNFß) sind zwei nahe verwandte Peptidfaktoren [3] aus der Klasse der Lymphokine/Cytokine, die im folgenden beide als TNF bezeichnet werden [siehe Uebersichtsarbeiten 2 und 3]. TNF verfügt über ein breites zelluläres Wirkungsspektrum. Beispielsweise besitzt TNF inhibierende oder cytotoxische Wirkung auf eine Reihe von Tumorzelllinien [2,3], stimuliert die Proliferation von Fibroblasten und die phagozytierende/cytotoxische Aktivität von myeloischen Zellen [4,5,6], induziert Adhäsionsmoleküle in Endothelzellen oder übt eine inhibierende Wirkung auf Endothel aus [7,8,9,10], inhibiert die Synthese von spezifischen Enzymen in Adipozyten [11] und induziert die Expression von Histokompatibilitätsantigenen [12]. Manche dieser TNF-Wirkungen werden über eine Induktion von anderen Faktoren oder durch synergistische Effekte mit anderen Faktoren, wie beispielsweise Interferonen oder Interleukinen erzielt [13-16].

TNF ist bei einer Reihe von pathologischen Zuständen, beispielsweise Schockzuständen bei Meningococcen-Sepsis [17], bei der Entwicklung von Autoimmun-Glomerulonephritis bei Mäusen [18] oder bei cerebraler Malaria bei Mäusen [19] und beim Menschen [41] involviert. Ganz allgemein scheinen die toxischen Wirkungen von Endotoxin durch TNF vermittelt zu sein [20]. Weiterhin kann TNF wie Interleukin-1 Fieber auslösen [39]. Auf Grund der pleiotropen funktionellen Eigenschaften von TNF kann man annehmen, dass TNF in Wechselwirkung mit anderen Cytokinen bei einer ganzen Reihe weiterer pathologischer Zustände als Mediator von Immunantwort, Entzündung oder anderen Prozessen beteiligt ist.

Diese biologischen Effekte werden durch TNF über spezifische Rezeptoren vermittelt, wobei nach heutigem Wissensstand sowohl TNFα wie TNFß an die gleichen Rezeptoren binden [21]. Verschiedene Zelltypen unterscheiden sich in der Anzahl von TNF-Rezeptoren [22,23,24]. Solche ganz allgemein gesprochen TNF-bindenden Proteine (TNF-BP) wurden durch kovalente Bindung an radioaktiv markiertes TNF nachgewiesen [24-29], wobei die folgenden scheinbaren Molekulargewichte der erhaltenen TNF/TNF-BP-Komplexe ermittelt wurden: 95/100 kD und 75 kD [24], 95 kD und 75 kD [25], 138 kD, 90 kD, 75 kD und 54 kD [26], 100±5 kD [27], 97 kD und 70 kD [28] und 145 kD [29]. Mittels anti-TNF-Antikörper-Immunoaffinitätschromatographie und präparativer SDS-Polyacrylamidgelelektrophorese (SDS-PAGE) konnte ein solcher TNF/TNF-BP-Komplex isoliert werden [27]. Die reduktive Spaltung dieses Komplexes und anschliessende SDS-PAGE-Analyse ergab mehrere Banden, die allerdings nicht auf TNF-Bindeaktivität getestet wurden. Da die spezifischen Bedingungen, die zu der Spaltung des Komplexes verwendet werden müssen, zur Inaktivierung des Bindeproteins führen [31], ist letzteres auch nicht möglich gewesen. Die Anreicherung von löslichen TNF-BP aus dem humanen Serum oder Urin mittels Ionenaustauscher-Chromatographie und Gelfiltration (Molekulargewichte im Bereich von 50 kD) wurde von Olsson et al. beschrieben [30].

Brockhaus et al. [32] erhielten durch TNFα-Ligandenaffinitätschromatographie und HPLC aus Membranextrakten von HL60-Zellen eine angereicherte TNF-BP-Präparation, die wiederum als Antigenpräparation zur Herstellung von monoklonalen Antikörpern gegen TNF-BP verwendet wurde. Unter Verwendung eines solchen immobilisierten Antikörpers (Immunaffinitätschromatographie) wurde mittels TNFα-Ligandenaffinitätschromatographie und HPLC von Loetscher und Brockhaus [31] aus einem Extrakt von humaner Placenta eine angereicherte Präparation von TNF-BP erhalten, die in der SDS-PAGE-Analyse eine starke breite Bande bei 35 kD, eine schwache Bande bei etwa 40 kD und eine sehr schwache Bande im Bereich zwischen 55 kD und 60 kD ergab. Im übrigen zeigte das Gel im Bereich von 33 kD bis 40 kD einen Proteinhintergrundschmier. Die Bedeutung der so erhaltenen Proteinbanden war jedoch im Hinblick auf die Heterogenität des verwendeten Ausgangsmaterials (Placenta-Gewebe; vereinigtes Material aus mehreren Placenten) nicht klar.

Gegenstand der vorliegenden Erfindung sind nichtlösliche Proteine, d.h. beispielsweise Membranproteine bzw. sogenannte Rezeptoren, und lösliche oder nichtlösliche Fragmente davon, die TNF binden (TNF-BP), in homogener Form, sowie deren physiologisch verträgliche Salze. Bevorzugt sind solche Proteine, die gemäss SDS-PAGE unter nicht reduzierenden Bedingungen durch scheinbare Molekulargewichte von etwa 55 kD, 51 kD, 38 kD, 36 kD und 34 kD bzw. 75 kD und 65 kD charakterisiert sind, insbesonders solche mit etwa 55 kD und 75 kD. Weiterhin bevorzugt sind solche Proteine, die durch wenigstens eine der folgenden Aminosäureteilsequenzen gekennzeichnet sind: 
(IA) Leu-Val-Pro-His-Leu-Gly-Asp-Arg-Glu-Lys-Arg-Asp-Ser-
Val-Cys-Pro-Gln-Gly-Lys-Tyr-Ile-His-Pro-Gln-X-Asn-Ser-
Ile
 
(IB) Ser-Thr-Pro-Glu-Lys-Glu-Gly-Glu-Leu-Glu-Gly-Thr-Thr-
Thr-Lys
(IIA) Ser-Gln-Leu-Glu-Thr-Pro-Glu-Thr-Leu-Leu-Gly-Ser-Thr-
Glu-Glu-Lys-Pro-Leu
(IIB) Val-Phe-Cys-Thr
(IIC) Asn-Gln-Pro-Gln-Ala-Pro-Gly-Val-Glu-Ala-Ser-Gly-Ala-
Gly-Glu-Ala
(IID) Leu-Pro-Ala-Gln-Val-Ala-Phe-X-Pro-Tyr-Ala-Pro-Glu-Pro-
Gly-Ser-Thr-Cys
(IIE) Ile-X-Pro-Gly-Phe-Gly-Val-Ala-Tyr-Pro-Ala-Leu-Glu
(IIF) Leu-Cys-Ala-Pro
(IIG) Val-Pro-His-Leu-Pro-Ala-Asp
(IIH) Gly-Ser-Gln-Gly-Pro-Glu-Gln-Gln-X-X-Leu-Ile-X-Ala-Pro
 wobei X für einen Aminosäurerest steht, der nicht eindeutig bestimmt werden konnte.

Im Stand der Technik sind bereits TNF-BP durch eine N-terminale Teilsequenz charakterisiert worden [Europäische Patentanmeldung mit der Publikations-Nr. 308 378], wobei sich diese Sequenz von der erfindungsgemässen N-terminalen Teilsequenz gemäss Formel (IA) unterscheidet. Im übrigen handelt es sich aber bei den im Stand der Technik beschriebenen TNF-Bindeproteinen um aus dem Urin isolierte, lösliche, d.h. nicht membrangebundene, TNF-BP und nicht um membrangebundene, d.h. unlösliche, TNF-BP.

Gegenstand der vorliegenden Anmeldung sind auch Verfahren zur Isolierung der erfindungsgemässen TNF-BP. Diese Verfahren sind dadurch charakterisiert, dass man im wesentlichen die folgenden Reinigungsschritte nacheinander ausführt: Herstellung eines Zell- oder Gewebeextraktes, Immunaffinitätschromatographie und/oder ein- oder mehrfache Ligandenaffinitätschromatographie, hochauflösende Flüssigkeitschromatographie (HPLC) und präparative SDS-Polyacrylamidgelelektrophorese (SDS-PAGE). Die Kombination der aus dem Stand der Technik bekannten einzelnen Reinigungsschritte ist für den Erfolg des erfindungsgemässen Verfahrens essentiell, wobei einzelne Schritte im Rahmen der zu lösenden Aufgabe modifiziert und verbessert wurden. So wurde beispielsweise der ursprünglich für die Anreicherung von TNF-BP aus humaner Placenta [31] verwendete kombinierte Immunaffinitätschromatographie/TNFα-Ligandenaffinitätschroma tographie-Schritt dadurch abgeändert, dass eine BSA-Sepharose 4B-Vorsäule verwendet wurde. Diese Vorsäule wurde zum Auftrag des Zell- oder Membranextraktes in Reihe mit der Immunaffinitätssäule und gefolgt von der Ligandenaffinitätssäule geschaltet. Nach Auftrag des Extraktes wurden die beiden zuletztgenannten Säulen abgekoppelt, jede für sich eluiert und die TNF-BP-aktiven Fraktionen wurden nochmals über eine Ligandenaffinitätssäule gereinigt. Erfindungswesentlich für die Durchführung des Umkehrphasen-HPLC--Schrittes ist die Verwendung eines Detergens-haltigen Lösungsmittelgemisches.

Ferner ist auch ein technisches Verfahren zum Erzielen hoher Zelldichten von Säugerzellen, aus denen TNF-BP isoliert werden können, Gegenstand der vorliegenden Erfindung. Ein solches Verfahren zeichnet sich dadurch aus, dass ein Medium, welches für die spezifischen Wachstumserfordernisse der verwendeten Zelllinie entwickelt wurde, in Verbindung mit einer wie z.B. im Detail in Beispiel 2 beschriebenen Perfusionsapparatur verwendet wird. Mittels eines solchen Verfahrens lassen sich beispielsweise für HL-60-Zellen bis zu mehr als 20-fach höhere Zelldichten als üblich erzielen.

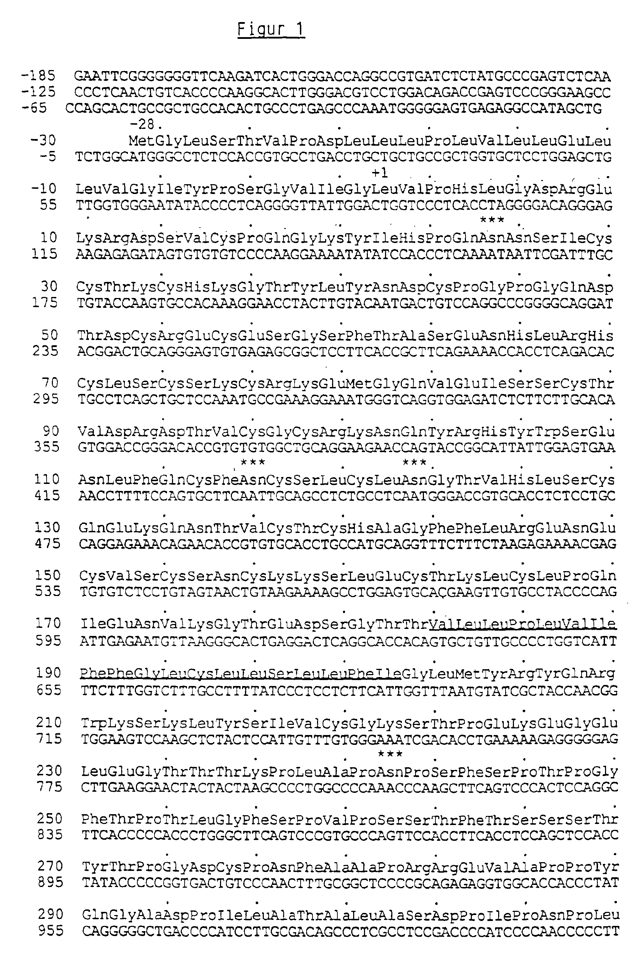

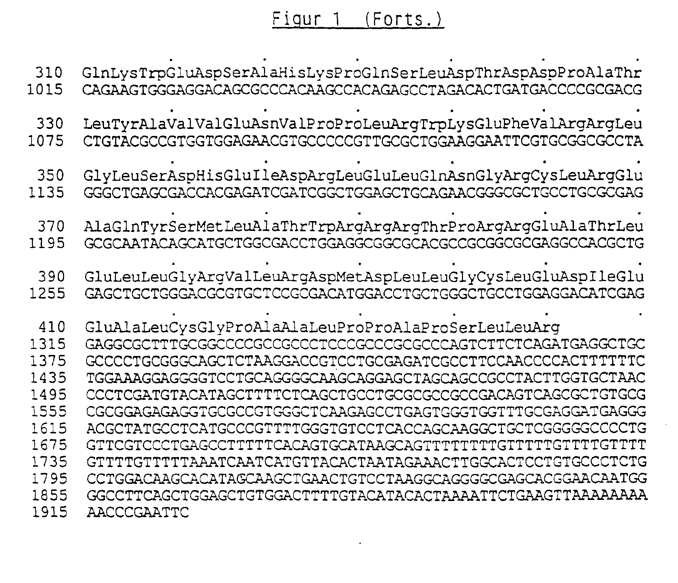

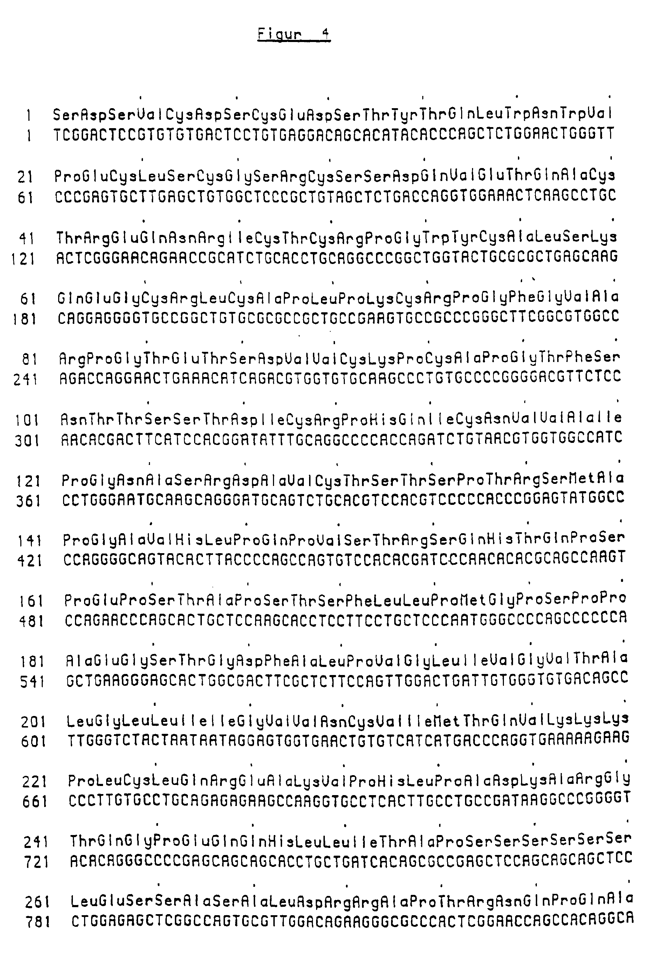

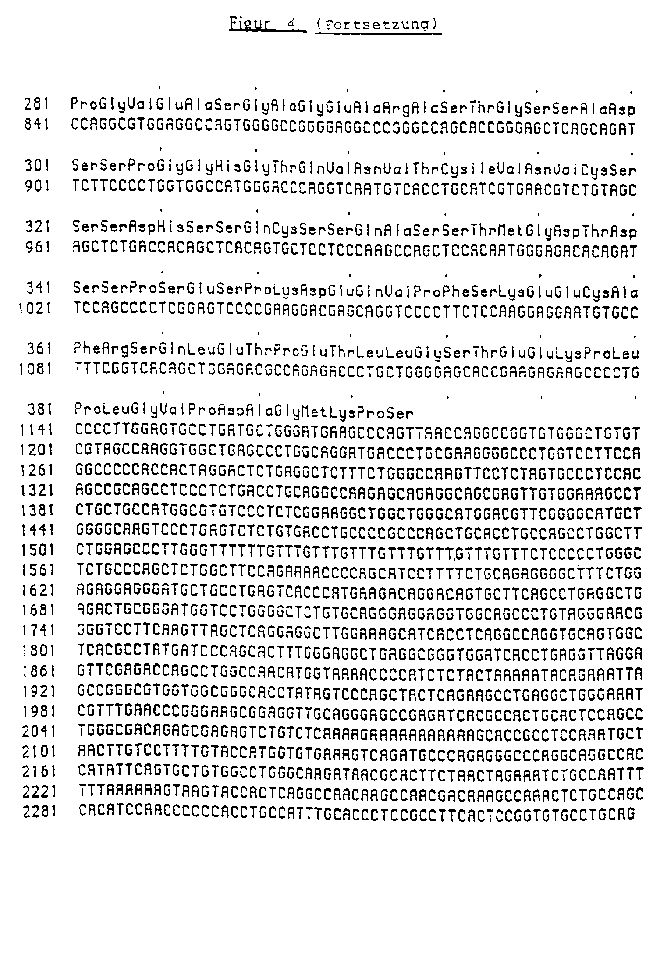

Zusätzlich dazu betrifft die vorliegende Erfindung auch DNA-Sequenzen, die für Proteine und lösliche oder nichtlösliche Fragmente davon, die TNF binden, kodieren. Darunter sind beispielsweise DNA-Sequenzen zu verstehen, die für nichtlösliche Proteine oder lösliche wie nicht lösliche Fragmente davon, die TNF binden, kodieren, wobei solche DNA-Sequenzen aus den folgenden auswählbar sind:
(a) DNA-Sequenzen, wie sie in Figur 1 oder Figur 4 dargestellt sind, wie deren komplementäre Stränge, oder solche, die diese Sequenzen umfassen;
(b) DNA-Sequenzen, die mit wie unter (a) definierten Sequenzen oder Fragmenten davon hybridisieren;
(c) DNA-Sequenzen, die auf Grund der Entartung des genetischen Codes nicht mit Sequenzen, wie unter (a) und (b) definiert, hybridisieren, aber die für Polypeptide mit genau gleicher Aminosäuresequenz kodieren.

Das heisst, dass von der vorliegenden Erfindung nicht nur allelische Varianten, sondern auch solche DNA-Sequenzen umfasst sind, die sich durch Deletionen, Substitutionen und Additionen von einem oder mehreren Nukleotiden der in Figur 1 bzw. 4 dargestellten Sequenzen ergeben, wobei es sich bei den davon kodierten Proteinen nach wie vor um TNF-BP handelt. Eine Sequenz, die sich durch eine solche Deletion ergibt, ist beispielsweise in Science 248, 1019-1023, (1990) beschrieben.

Bevorzugt sind einmal solche DNA-Sequenzen, welche für ein solches Protein mit einem scheinbaren Molekulargewicht von etwa 55 kD kodieren, wobei die in Abbildung 1 dargestellte Sequenz besonders bevorzugt ist, wie Sequenzen, die für nichtlösliche wie lösliche Fragmente von solchen Proteinen kodieren. Eine DNA-Sequenz, die beispielsweise für ein solches nichtlösliches Protein-Fragment kodiert, reicht von Nukleotid -185 bis 1122 der in Abbildung 1 gezeigten Sequenz. DNA-Sequenzen, die für lösliche Protein-Fragmente kodieren, sind beispielsweise solche, die von Nukleotid -185 bis 633 bzw. von Nukleotid -14 bis 633 der in Abbildung 1 gezeigten Sequenz reichen. Bevorzugt sind ebenfalls DNA-Sequenzen, die für ein Protein von etwa 75/65 kD kodieren, wobei solche, die die in Figur 4 dargestellte partielle cDNA-Sequenzen enthalten, bevorzugt sind. Besonders bevorzugte DNA-Sequenzen sind in diesem Fall die Sequenzen des offenen Leserasters von Nukleotid 2 bis 1'177. Die Peptide IIA, IIC, IIE, IIF, IIG und IIH werden von der partiellen cDNA-Sequenz in Figur 4 kodiert, wobei die geringfügigen Abweichungen der experimentell bestimmten Aminosäuresequenzen von der von der cDNA abgeleiteten Sequenz mit höchster Wahrscheinlichkeit auf der geringeren Auflösung der Gasphasen-Sequenzierung beruhen. Bevorzugt sind auch DNA-Sequenzen, die für nichtlösliche wie lösliche Fragmente von TNF-bindenden Proteinen mit einem scheinbaren Molekulargewicht von 75 kD/65 kD kodieren. DNA-Sequenzen für solche löslichen Fragmente können auf Grund der Hydrophilie-profile der von den für solche nichtlöslichen TNF-BP kodierenden Nukleinsäuresequenzen abgeleiteten Aminosäuresequenzen bestimmt werden.

Die Erfindung betrifft weiterhin DNA-Sequenzen, die eine Kombination aus zwei Teil-DNA-Sequenzen umfassen, wobei die eine Teilsequenz für solche löslichen Fragmente von nichtlöslichen Proteinen, die TNF binden kodiert (s.o.) und die andere Teil-Sequenz, für alle Domänen ausser der ersten Domäne der konstanten Region der schweren Kette von humanen Immunglobulinen, wie IgG, IgA, IgM bzw. IgE, kodiert.

Die vorliegende Erfindung betrifft natürlich auch die von solchen DNA-Sequenzen kodierten rekombinanten Proteine. Selbstverständlich sind dabei auch solche Proteine umfasst, in deren Aminosäuresequenzen, beispielsweise mittels gezielter Mutagenese, Aminosäuren so ausgetauscht worden sind, dass dadurch die Aktivität der TNF-BP oder deren Fragmente, nämlich die Bindung von TNF oder die Wechselwirkung mit anderen, an der Signalübertragung beteiligten Membrankomponenten, in einer gewünschten Art verändert oder erhalten wurden. Aminosäureaustausche in Proteinen und Peptiden, die im allgemeinen die Aktivität solcher Moleküle nicht verändern, sind im Stand der Technik bekannt und beispielsweise von H. Neurath und R.L. Hill in "The Proteins" (Academic Press, New York, 1979, siehe besonders Figur 6, Seite 14) beschrieben. Die am häufigsten vorkommenden Austausche sind: Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly, sowie solche in umgekehrter Weise. Die vorliegende Erfindung betrifft ferner Vektoren, die erfindungsgemässe DNA-Sequenzen enthalten und zur Transformation von geeigneten pro- wie eukaryotischen Wirtssystemen geeignet sind, wobei solche Vektoren bevorzugt sind, deren Verwendung zur Expression der von den erfindungsgemässen DNA-Sequenzen kodierten Proteine führt. Schliesslich betrifft die vorliegende Erfindung auch noch mit solchen Vektoren transformierte pro- wie eukaryotische Wirtssysteme, wie Verfahren zur Herstellung von erfindungsgemässen rekombinanten Verbindungen durch Kultivierung solcher Wirtssysteme und anschliessende Isolierung dieser Verbindungen aus den Wirtssystemen selbst oder deren Kulturüberständen.

Gegenstand der vorliegenden Erfindung sind auch pharmazeutische Präparate, die wenigstens eines dieser TNF-BP oder Fragmente davon, gewünschtenfalls in Verbindung mit weiteren pharmazeutisch wirksamen Substanzen und/oder nicht-toxischen, inerten, therapeutisch verträglichen Trägermaterialien enthalten.

Die vorliegende Erfindung betrifft schliesslich die Verwendung solcher TNF-BP einerseits zur Herstellung pharmazeutischer Präparate bzw. andererseits zur Behandlung von Krankheiten, bevorzugt solchen, in deren Verlauf TNF involviert ist.

Ausgangsmaterial für die erfindungsgemässen TNF-BP sind ganz allgemein Zellen, die solche TNF-BP in membrangebundener Form enthalten und die dem Fachmann ohne Beschränkungen allgemein zugänglich sind, wie beispielsweise HL60- [ATCC Nr. CCL 240], U 937- [ATCC Nr. CRL 1593], SW 480- [ATCC Nr. CCL 228] und HEp2-Zellen [ATCC Nr. CCL 23]. Diese Zellen können nach bekannten Methoden des Standes der Technik [40] oder zum Erzielen hoher Zelldichten nach dem bereits allgemein und im Detail für HL60-Zellen in Beispiel 2 beschriebenen Verfahren kultiviert werden. TNF-BP können dann nach bekannten Methoden des Standes der Technik mittels geeigneter Detergenzien, beispielsweise Triton X-114, 1-0-n-Octyl--β-D-glucopyranosid (Octylglucosid), oder 3-[(3-Cholylamidopropyl)-dimethylammonio]-1-propansulfonat (CHAPS), im besonderen mittels Triton X-100, aus den aus dem Medium abzentrifugierten und gewaschenen Zellen extrahiert werden. Zum Nachweis solcher TNF-BP können die üblicherweise verwendeten Nachweismethoden für TNF-BP, beispielsweise eine Polyäthylenglykol-induzierte Fällung des ¹²⁵I-TNF/TNF-BP-Komplexes [27], im besonderen Filterbindungstests mit radioaktiv markiertem TNF gemäss Beispiel 1, verwendet werden. Zur Gewinnung der erfindungsgemässen TNF-BP können die generell zur Reinigung von Proteinen, insbesondere von Membranproteinen, verwendeten Methoden des Standes der Technik, wie beispielsweise Ionenaustausch-Chromatographie, Gelfiltration, Affinitätschromatographie, HPLC und SDS-PAGE verwendet werden. Besonders bevorzugte Methoden zur Herstellung erfindungsgemässer TNF-BP sind Affinitätschromatographie, insbesondere mit TNF-α als an die Festphase gebundenen Liganden und Immunaffinitätschromatographie, HPLC und SDS-PAGE. Die Elution von mittels SDS-PAGE aufgetrennten TNF-BP Banden kann nach bekannten Methoden der Proteinchemie erfolgen, beispielsweise mittels Elektroelution nach Hunkapiller et al. [34], wobei nach heutigem Stand des Wissens die dort angegebenen Elektro-Dialysezeiten generell zu verdoppeln sind. Danach noch verbleibende Spuren von SDS können dann gemäss Bosserhoff et al. [50] entfernt werden.

Die so gereinigten TNF-BP können mittels der im Stand der Technik bekannten Methoden der Peptidchemie, wie beispielsweise N-terminale Aminosäuresequenzierung oder enzymatische wie chemische Peptidspaltung charakterisiert werden. Durch enzymatische oder chemische Spaltung erhaltene Fragmente können nach gängigen Methoden, wie beispielsweise HPLC, aufgetrennt und selbst wieder N-terminal sequenziert werden. Solche Fragmente, die selbst noch TNF binden, können mittels der obengenannten Nachweismethoden für TNF-BP identifiziert werden und sind ebenfalls Gegenstand der vorliegenden Erfindung.

Ausgehend von der so erhältlichen Aminosäuresequenzinformation oder den in Figur 1 wie Figur 4 dargestellten DNA- wie Aminosäuresequenzen können unter Beachtung der Degeneration des genetischen Codes nach im Stand der Technik bekannten Methoden geeignete Oligonukleotide hergestellt werden [51]. Mittels dieser können dann wiederum nach bekannten Methoden der Molekularbiologie [42,43] cDNA- oder genomische DNA-Banken nach Klonen, die für TNF-BP kodierende Nukleinsäuresequenzen enthalten, abgesucht werden. Ausserdem können mittels der Polymerase-Kettenreaktion (PCR) [49] cDNA-Fragmente kloniert werden, indem von zwei auseinanderliegenden, relativ kurzen Abschnitten der Aminosäuresequenz unter Beachtung des genetischen Codes vollständig degenerierte und in ihrer Komplementarität geeignete Oligonucleotide als "Primer" eingesetzt werden, wodurch das zwischen diesen beiden Sequenzen liegende Fragment amplifiziert und identifiziert werden kann. Die Bestimmung der Nukleotidsequenz eines derartigen Fragmentes ermöglicht eine unabhängige Bestimmung der Aminosäure-Sequenz des Protein-fragments, für das es kodiert. Die mittels der PCR erhältlichen cDNA-Fragmente können ebenfalls, wie bereits für die Oligonukleotide selbst beschrieben, nach bekannten Methoden zum Aufsuchen von für TNF-BP kodierende Nukleinsäuresequenzen enthaltenden Klonen aus cDNA- bzw. genomische DNA-Banken verwendet werden. Solche Nukleinsäuresequenzen können dann nach bekannten Methoden sequenziert werden [42]. Aufgrund der so bestimmten wie der für bestimmte Rezeptoren bereits bekannten Sequenzen, können solche Teilsequenzen, die für lösliche TNF-BP-Fragmente kodieren, bestimmt und mittels bekannter Methoden aus der Gesamtsequenz herausgeschnitten werden [42].

Die gesamte Sequenz oder solche Teilsequenzen können dann mittels bekannter Methoden in im Stand der Technik beschriebene Vektoren zu deren Vervielfältigung wie Expression in Prokaryoten integriert werden [42]. Geeignete prokaryotische Wirtsorganismen stellen beispielsweise gram-negative wie gram-positive Bakterien, wie beispielsweise E. coli Stämme, wie E. coli HB 101 [ATCC Nr. 33 694] oder E. coli W3110 [ATCC Nr. 27 325] oder B. subtilis Stämme dar.

Weiterhin können erfindungsgemässe Nukleinsäuresequenzen, die für TNF-BP sowie für TNF-BP-Fragmente kodieren, in geeignete Vektoren zur Vermehrung wie Expression in eukaryotischen Wirtszellen, wie beispielsweise Hefe, Insekten- und Säugerzellen, mittels bekannter Methoden integriert werden. Expression solcher Sequenzen erfolgt bevorzugt in Säuger- wie Insektenzellen.

Ein typischer Expressionsvektor für Säugerzellen enthält ein effizientes Promotorelement, um eine gute Transkriptionsrate zu erzielen, die zu exprimierende DNA-Sequenz und Signale für eine effiziente Termination und Polyadenylierung des Transkripts. Weitere Elemente, die verwendet werden können, sind "Enhancer", welche zu nochmals verstärkter Transkription führen und Sequenzen, welche z.B. eine längere Halbwertszeit der mRNA bewirken können. Zur Expression von Nukleinsäuresequenzen, denen das endogene für ein Signalpeptid kodierende Sequenzstück fehlt, können Vektoren verwendet werden, die solche geeignete Sequenzen, die für Signalpeptide von anderen bekannten Proteinen kodieren, enthalten. Siehe beispielsweise der von Cullen, B.R. in Cell 46, 973-982 (1986) beschriebene Vektor pLJ268 oder auch bei Sharma, S. et al. in "Current Communications in Molecular Biology", edt. by Gething, M.J., Cold Spring Harbor Lab. (1985), Seiten 73-78.

Die meisten Vektoren, die für eine transiente Expression einer bestimmten DNA-Sequenz in Säugerzellen verwendet werden, enthalten den Replikationsursprung des SV40 Virus. In Zellen, die das T-Antigen des Virus exprimieren, (z.B. COS-Zellen), werden diese Vektoren stark vermehrt. Eine vorübergehende Expression ist aber nicht auf COS-Zellen beschränkt. Im Prinzip kann jede transfektierbare Säugerzelllinie hierfür verwendet werden. Signale, die eine starke Transkription bewirken können, sind z.B. die frühen und späten Promotoren von SV40, der Promoter und Enhancer des "major immediate-early" Gens des HCMV (humaner Cytomegalovirus), die LTRs ("long terminal repeats") von Retroviren, wie beispielsweise RSV, HIV und MMTV. Es können aber auch Signale von zellulären Genen, wie z.B. die Promotoren des Aktin- und Collagenase-Gens, verwendet werden.

Alternativ können aber auch stabile Zelllinien, die die spezifische DNA-Sequenz im Genom (Chromosom) integriert haben, erhalten werden. Hierzu wird die DNA-Sequenz zusammen mit einem selektierbaren Marker, z.B. Neomycin, Hygromycin, Dihydrofolat-Reduktase (dhfr) oder Hypoxanthin-Guanin-Phosphoribosyltransferase (hgpt) kotransfektiert. Die stabil ins Chromosom eingebaute DNA-Sequenz kann auch noch stark vermehrt werden. Ein geeigneter Selektionsmarker hierfür ist beispielsweise die Dihydrofolat-Reduktase (dhfr). Säugerzellen (z.B. CHO-Zellen), welche kein intaktes dhfr-Gen enthalten, werden hierbei nach erfolgter Transfektion mit steigenden Mengen von Methotrexat inkubiert. Auf diese Weise können Zelllinien erhalten werden, welche mehr als tausend Kopien der gewünschten DNA-Sequenz enthalten.

Säugerzellen, welche für die Expression verwendet werden können, sind z.B. Zellen der menschlichen Zelllinien Hela [ATCC CCL2] und 293 [ATCC CRL 1573], sowie 3T3- [ATCC CCL 163] und L-Zellen, z.B. [ATCC CCL 149], (CHO)-Zellen [ATCC CCL 61], BHK [ATCC CCL 10]-Zellen sowie die CV 1 [ATCC CCL 70]- und die COS-Zelllinien [ATCC CRL 1650, CRL 1651].

Geeignete Expressionsvektoren umfassen beispielsweise Vektoren wie pBC12MI [ATCC 67 109], pSV2dhfr [ATCC 37 146], pSVL [Pharmacia, Uppsala, Sweden], pRSVcat [ATCC 37 152] und pMSG [Pharmacia, Uppsala, Sweden]. Besonder bevorzugte Vektoren sind die in Beispiel 9 verwendeten Vektoren "pK19" und "pN123". Diese können aus den mit ihnen transformierten E. coli-Stämmen HB101(pK19) und HB101(pN123) nach bekannten Methoden isoliert werden [42]. Diese E. coli-Stämme wurden am 26. Januar 1990 bei der Deutschen Sammlung von Mikroorganismen und Zellkulturen GmbH (DSM) in Braunschweig, BRD unter DSM 5761 für HB101(pK19) und DMS 5764 für HB101(pN123) hinterlegt. Zur Expression der Proteine, die aus einem löslichen Fragment von nichtlöslichen TNF-BP und einem Immunglobulinanteil, d.h. allen Domänen ausser der ersten der konstanten Region der schweren Kette, bestehen, eignen sich besonders pSV2 abgeleitete Vektoren wie beispielsweise von German, C. in "DNA Cloning" [Vol. II., edt von Glover, D.M., IRL Press, Oxford, 1985] beschrieben. Besonders bevorzugte Vektoren sind die bei der Deutschen Sammlung von Mikroorganismen und Zellkulturen GmbH (DSM) in Braunschweig, BRD hinterlegten und in der Europäischen Patentanmeldung Nr. 90107393.2 genau beschriebenen Vektoren pCD4-Hµ (DSM 5315), pCD4-Hγl (DSM 5314) und pCD4-Hγ3 (DSM 5523). Besagte Europäische Patentschrift wie die in Beispiel 11 angegebenen äquivalenten Anmeldungen enthalten auch Angaben bezüglich der weiteren Verwendung dieser Vektoren zur Expression von chimären Proteinen (siehe auch Beispiel 11) wie zur Konstruktion von Vektoren für die Expression von solchen chimären Proteinen mit anderen Immunglobulinanteilen.

Die Art und Weise wie die Zellen transfektiert werden hängt vom gewählten Expressions- und Vektorsystem ab. Eine Uebersicht über diese Methoden findet man z.B. bei Pollard et al., "DNA Transformation of Mammalian Cells" in "Methods in Molecular Biology" [Nucleic Acids Vol. 2, 1984, Walker, J.M., ed, Humana, Clifton, New Jersey]. Weitere Methoden findet man bei Chen und Okayama ["High-Efficiency Transformation of Mammalian Cells by Plasmid DNA", Molecular and Cell Biology 7, 2745-2752, 1987] und bei Felgner [Felgner et al., "Lipofectin: A highly efficient, lipid-mediated DNA-transfection procedure", Proc. Nat. Acad. Sci. USA 84, 7413-7417, 1987].

Zur Expression in Insektenzellen kann das Baculovirus--Expressions-System, welches schon für die Expression einer Reihe von Proteinen erfolgreich eingesetzt worden ist (für eine Uebersicht siehe Luckow and Summers, Bio/Technology 6, 47-55, 1988), verwendet werden. Rekombinante Proteine können authentisch oder als Fusionsproteine hergestellt werden. Die so hergestellten Proteine können auch modifiziert, wie beispielsweise glykosyliert (Smith et al., Proc. Nat. Acad. Sci. USA 82, 8404-8408, 1987) sein. Für die Herstellung eines rekombinanten Baculovirus, der das gewünschte Protein exprimiert, verwendet man einen sogenannten "Transfervektor". Hierunter versteht man ein Plasmid, welches die heterologe DNA-Sequenz unter der Kontrolle eines starken Promoters, z.B. dem des Polyhedringens, enthält, wobei diese auf beiden Seiten von viralen Sequenzen umgeben ist. Besonders bevorzugte Vektoren sind die in Beispiel 10 verwendeten Vektoren "pN113", "pN119" und "pN124". Diese können aus den mit ihnen transformierten E. coli-Stämmen HB101(pN113), HB101(pN119) und HB101(pN124) nach bekannten Methoden isoliert werden [42]. Diese E. coli-Stämme wurden am 26. Januar 1990 bei der Deutschen Sammlung von Mikroorganismen und Zellkulturen GmbH (DSM) in Braunschweig, BRD, unter DSM 5762 für HB101(pN113), DSM 5763 für HB101(pN119) und DSM 5765 für HB101(pN124) hinterlegt. Der Transfervektor wird dann zusammen mit DNA des Wildtyp-Baculovirus in die Insektenzellen transfektiert. Die in den Zellen durch homologe Rekombination entstehenden rekombinanten Viren können dann nach bekannten Methoden identifiziert und isoliert werden. Eine Uebersicht über das Baculovirus-Expressionssystem und der dabei verwendeten Methoden findet man bei Luckow und Summers [52].

Exprimierte TNF-BP wie ihre nichtlöslichen oder löslichen Fragmente können dann nach im Stand der Technik bekannten Methoden der Proteinchemie, wie beispielsweise den bereits auf Seiten 5-6 beschriebenen Verfahren, aus der Zellmasse oder den Kulturüberständen gereinigt werden.

Die erfindungsgemäss erhaltenen TNF-BP können auch als Antigene zur Erzeugung von poly- und monoklonalen Antikörpern nach bekannten Methoden der Technik [44,45] oder gemäss dem in Beispiel 3 beschriebenen Verfahren verwendet werden. Solche Antikörper, insbesondere monoklonale Antikörper gegen die 75 kD-TNF-BP-Spezies, sind ebenfalls Gegenstand der vorliegenden Erfindung. Solche gegen die 75 kD TNF-BP gerichtete Antikörper können durch dem Fachmann geläufige Modifikationen des in den Beispielen 4-6 im Detail beschriebenen Reinigungsverfahrens zur Isolierung von TNF-BP eingesetzt werden.

Auf Grund der hohen Bindungsaffinität erfindungsgemässer TNF-BP für TNF (K_{d}-Werte in den Grössenordnungen von 10⁻⁹ - 10⁻¹⁰M) können diese oder Fragmente davon als Diagnostika zum Nachweis von TNF in Serum oder anderen Körperflüssigkeiten nach im Stand der Technik bekannten Methoden, beispielsweise in Festphasenbindungstests oder in Verbindung mit Anti-TNF-BP-Antikörpern in sogenannten "Sandwich"-Tests, eingesetzt werden.

Im übrigen können erfindungsgemässe TNF-BP einerseits zur Reinigung von TNF und andererseits zum Auffinden von TNF-Agonisten sowie TNF-Antagonisten nach im Stand der Technik bekannten Verfahren verwendet werden.

Die erfindungsgemässen TNF-BP sowie deren physiologisch verträgliche Salze, die nach im Stand der Technik bekannten Methoden hergestellt werden können, können auch zur Herstellung von pharmazeutischen Präparaten, vor allem solchen zur Behandlung von Krankheiten, bei deren Verlauf TNF involviert ist, verwendet werden. Dazu kann eine oder mehrere der genannten Verbindungen, falls wünschenswert bzw. erforderlich in Verbindung mit anderen pharmazeutisch aktiven Substanzen, mit den üblicherweise verwendeten festen oder flüssigen Trägermaterialien in bekannter Weise verarbeitet werden. Die Dosierung solcher Präparate kann unter Berücksichtigung der üblichen Kriterien in Analogie zu bereits verwendeten Präparaten ähnlicher Aktivität und Struktur erfolgen.

Nachdem die Erfindung vorstehend allgemein beschrieben worden ist, sollen die folgenden Beispiele Einzelheiten der Erfindung veranschaulichen, ohne dass diese dadurch in irgendeiner Weise eingeschränkt wird.

### Beispiel 1

### Nachweis von TNF-bindenden Proteinen

Die TNF-BP wurden in einem Filtertest mit humanem radio-jodiertem ¹²⁵I-TNF nachgewiesen. TNF (46,47) wurde mit Na¹²⁵ I (IMS40, Amersham, Amersham, England) und Iodo-Gen (#28600, Pierce Eurochemie, Oud-Beijerland, Niederlande) nach Fraker und Speck [48] radioaktiv makiert. Zum Nachweis der TNF-BP wurden isolierte Membranen der Zellen oder ihre solubilisierten, angereicherten und gereinigten Fraktionen auf angefeuchtete Nitrocellulose-Filter (0.45 µ, BioRad, Richmond, California, USA) aufgetragen. Die Filter wurden dann in Pufferlösung mit 1% entfettetem Milchpulver blockiert und anschliessend mit 5•10⁵ cpm/ml ¹²⁵I-TNFα (0.3-1.0•10⁸ cpm/µg) in zwei Ansätzen mit und ohne Beigabe von 5µg/ml nicht-markiertem TNFα inkubiert, gewaschen und luftgetrocknet. Die gebundene Radioaktivität wurde autoradiographisch semiquantitativ nachgewiesen oder in einem γ-Counter gezählt. Die spezifische ¹²⁵I-TNF-α-Bindung wurde nach Korrektur für unspezifische Bindung in Anwesenheit von unmarkiertem TNF-α im Ueberschuss ermittelt. Die spezifische TNF-Bindung im Filtertest wurde bei verschiedenen TNF-Konzentrationen gemessen und nach Scatchard analysiert [33], wobei ein K_{d}-Wert von ~10⁻⁹ -10⁻¹⁰M ermittelt wurde.

### Beispiel 2

### Zellextrakte von HL-60-Zellen

HL60 Zellen [ATCC-Nr. CCL 240] wurden in experimentellem Labormasstab in einem RPMI 1640-Medium [GIBCO-Katalog Nr. 074-01800], das noch 2 g/l NaHCO₃ und 5% fötales Kälberserum enthielt, in einer 5% CO₂-Atmosphäre kultiviert und anschliessend zentrifugiert.

Zum Erzielen hoher Zelldichten in technischem Masstab wurde folgendermassen verfahren. Die Züchtung wurde in einem 75 1 Airliftfermenter (Fa. Chemap, Schweiz) mit 58 1 Arbeitsvolumen durchgeführt. Hierfür wurde das Kassetten-membransystem "PROSTAK" (Millipore, Schweiz) mit einer Membranfläche von 0,32 m² (1 Kassette) in den äusseren Zirkulationskreislauf integriert. Das Kulturmedium (siehe Tabelle 1) wurde mit einer Watson-Marlow Pumpe, Typ 603U, mit 5 l/min. umgepumpt. Nach einer Dampfsterilisation der Anlage, wobei das "PROSTAK" System im Autoklaven separat sterilisiert wurde, wurde die Fermentation mit wachsenden HL-60 Zellen aus einem 20 1 Airliftfermenter (Chemap) gestartet. Die Zellzüchtung im Impffermenter erfolgte im konventionellen Batchverfahren in dem Medium gemäss Tabelle 1 und einem Startzelltiter von 2x10⁵ Zellen/ml. Nach 4 Tagen wurde der HL60 Ansatz mit einem Titer von 4,9x10⁶ Zellen/ml in den 75 l Fermenter überführt. Der pH-Wert wurde bei 7,1 und der pO₂ Wert bei 25% Sättigung gehalten, wobei der Sauerstoffeintrag durch eine mikroporöse Fritte erfolgte. Nach anfänglicher Batchfermentation wurde am 2. Tag die Perfusion bei einem Zelltiter von 4x10⁶ Zellen/ml mit 30 1 Mediumsaustausch pro Tag gestartet. Auf der Filtratseite der Membran wurde das konditionierte Medium abgezogen und durch den Zulauf von frischem Medium ersetzt. Das Zulaufmedium wurde wie folgt verstärkt: Primatone von 0,25% auf 0.35%, Glutamin von 5 mM auf 6 mM und Glucose von 4 g/l auf 6 g/l. Die Perfusionsrate wurde dann am 3. und 4. Tag auf 72 l Medium/Tag und am 5. Tag auf 100 l Medium/Tag erhöht. Nach 120 Stunden der kontinuierlichen Züchtung wurde die Fermentation beendet. Unter den gegebenen Fermentationsbedingungen erfolgte exponentielles Zellwachstum bis 40x10⁶ Zellen/ml. Die Verdopplungszeit der Zellpopulation betrug bis 10x10⁶ Zellen/ml 20-22 Stunden und stieg dann mit zunehmender Zelldichte auf 30-36 Stunden an. Der Anteil der lebenden Zellen lag während der gesamten Fermentationszeit bei 90-95%. Der HL-60 Ansatz wurde dann im Fermenter auf ca. 12°C heruntergekühlt und die Zellen durch Zentrifugation (Beckman-Zentrifuge [Modell J-6B, Rotor JS], 3000 rpm, 10 min., 4°C) geerntet.

**Tabelle 1**

| HL-60 Medium | |
|---|---|
| Komponenten | Konzentrationen mg/l |
| CaCl₂ (wasserfrei) | 112,644 |
| Ca(NO₃)₂•4H₂O | 20 |
| CUSO₄•5H₂O | 0,498•10⁻³ |
| Fe(NO₃)₃•9H₂O | 0,02 |
| FeSO₄•7H₂O | 0,1668 |
| KCl | 336,72 |
| KNO₃ | 0.0309 |
| MgCl₂ (wasserfrei) | 11,444 |
| MgSO₄ (wasserfrei) | 68,37 |
| NaCl | 5801,8 |
| Na₂HPO₄ (wasserfrei) | 188,408 |
| NaH₂PO₄•H₂O | 75 |
| Na₂SeO₃•5H₂O | 9,6•10⁻³ |
| ZnSO₄•7H₂O | 0,1726 |
| | |
| D-Glucose | 4000 |
| Glutathion (red.) | 0,2 |
| Hepes-Puffer | 2383,2 |
| Hypoxanthin | 0,954 |
| Linolsäure | 0,0168 |
| Liponsäure | 0,042 |
| Phenolrot | 10,24 |
| Putrescin 2HCl | 0,0322 |
| Na-Pyruvat | 88 |
| Thymidin | 0,146 |
| Biotin | 0,04666 |
| D-Ca-Pantothenat | 2,546 |
| Cholinchlorid | 5,792 |
| Folsäure | 2,86 |
| i-Inositol | 11,32 |
| Niacinamid | 2,6 |
| Nicotinamid | 0,0074 |
| para-Aminobenzoesäure | 0,2 |
| Pyridoxal HCl | 2,4124 |
| Pyridoxin HCl | 0,2 |
| Riboflavin | 0,2876 |
| Thiamin HCl | 2,668 |
| Vitamin B₁₂ | 0,2782 |
| | |
| L-Alanin | 11,78 |
| L-Asparaginsäure | 10 |
| L-Asparagin H₂O | 14,362 |
| L-Arginin | 40 |
| L-Arginin HCl | 92,6 |
| L-Aspartat | 33,32 |
| L-Cystin 2HCl | 62,04 |
| L-Cystein HCl•H₂O | 7,024 |
| L-Glutaminsäure | 36,94 |
| L-Glutamin | 730 |
| L-Glycin | 21,5 |
| L-Histidin | 3 |
| L-Histidin HCl•H₂O | 27,392 |
| L-Hydroxypyrolin | 4 |
| L-Isoleucin | 73,788 |
| L-Leucin | 75,62 |
| L-Lysin HCl | 102,9 |
| L-Methionin | 21,896 |
| L-Phenylalanin | 43,592 |
| L-Prolin | 26,9 |
| L-Serin | 31,3 |
| L-Threonin | 53 |
| L-Tryptophan | 11,008 |
| L-Tyrosin•2Na | 69,76 |
| L-Valin | 62,74 |
| | |
| Penicillin/Streptomycin | 100 U/ml |
| Insulin (human) | 5 µg/ml |
| Tranferrin (human) | 15 µg/ml |
| Rinderserumalbumin | 67 µg/ml |
| Primatone RL (Sheffield Products, Norwich NY, USA) | 0,25% |
| Pluronic F68 (Serva, Heidelberg, BRD) | 0,01% |
| Fötales Kälberserum | 0,3-3% |

Das Zentrifugat wurde mit isotonem Phosphatpuffer (PBS: 0,2 g/l KCl. 0,2 g/l KH₂PO₄, 8,0 g/l NaCl, 2,16 g/l Na₂HPO₄ • 7H₂O), der mit 5% Dimethylformamid, 10 mM Benzamidin, 100 E/ml Aprotinin, 10 µM Leupeptin, 1 µM Pepstatin, 1 mM o-Phenanthrolin, 5 mM Jodacetamid, 1 mM Phenylmethylsulfonylfluorid versetzt war (im folgenden als PBS-M bezeichnet), gewaschen. Die gewaschenen Zellen wurden bei einer Dichte von 2,5•10⁸ Zellen/ml in PBS-M mit Triton X-100 (Endkonzentration 1,0%) extrahiert. Der Zellextrakt wurde durch Zentrifugation geklärt (15'000 x g, 1 Stunde; 100'000 x g, 1 Stunde).

### Beispiel 3

### Herstellung von monoklonalen (TNF-BP)-Antikörpern

Ein gemäss Beispiel 2 erhaltener Zentrifugationsüberstand aus Kultivierung von HL60-Zellen im experimentellen Labormasstab wurde im Verhältnis 1:10 mit PBS verdünnt. Der verdünnte Ueberstand wurde bei 4°C auf eine Säule aufgetragen (Flussrate: 0,2 ml/min.), die 2 ml Affigel 10 enthielt (Bio Rad Katalog Nr. 153-6099), an das 20 mg rekombinantes humanes TNF-α [Pennica, D. et al. (1984) Nature 312, 724; Shirai, T. et al. (1985) Nature 313, 803; Wang, A.M. et al. (1985) Science 228, 149] gemäss den Empfehlungen des Herstellers gekoppelt worden war. Die Säule wurde bei 4°C und einer Durchflussrate von 1 ml/min zuerst mit 20 ml PBS, das 0.1% Triton X 114 enthielt und danach mit 20 ml PBS gewaschen. So angereichertes TNF-BP wurde bei 22°C und einer Flussrate von 2 ml/min mit 4 ml 100 mM Glycin, pH 2.8, 0,1% Decylmaltosid eluiert. Das Eluat wurde in einer Centricon 30 Einheit [Amicon] auf 10 µl konzentriert.

10 µl dieses Eluates wurden mit 20 µl vollständigem Freundschen Adjuvans zu einer Emulsion gemischt. Je 10 µl der Emulsion wurden gemäss dem von Holmdahl, R. et al. [(1985), J. Immunol. Methods 83, 379] beschriebenen Verfahren an den Tagen 0, 7 und 12 in eine hintere Fusspfote einer narkotisierten Balb/c-Maus injiziert.

Am Tag 14 wurde die immunisierte Maus getötet, der popliteale Lymphknoten herausgenommen, zerkleinert und in Iscove's Medium (IMEM, GIBCO Katalog Nr. 074-2200), das 2 g/l NaHCO₃ enthielt, durch wiederholtes Pipettieren suspendiert. Gemäss einem modifizierten Verfahren von De St.Groth und Scheidegger [J. Immunol. Methods (1980), 35, 1] wurden 5x10⁷ Zellen des Lymphknotens mit 5x10⁷ PAI Maus--Myelomazellen (J.W. Stocker et al., Research Disclosure, 217, Mai 1982, 155-157), die sich in logarithmischem Wachstum befanden, fusioniert. Die Zellen wurden gemischt, durch Zentrifugation gesammelt und durch leichtes Schütteln in 2 ml 50% (v/v) Polyethylenglycol in IMEM bei Raumtemperatur resuspendiert und durch langsame Zugabe von 10 ml IMEM während 10 Minuten vorsichtigen Schüttelns verdünnt. Die Zellen wurden durch Zentrifugation gesammelt und in 200 ml vollständigem Medium [IMEM + 20% fötales Kälberserum, Glutamin (2,0 mM), 2-Mercaptoethanol (100 µM), 100 µM Hypoxanthine, 0,4 µM Aminopterine und 16 µM Thymidine (HAT)] resuspendiert. Die Suspension wurde auf 10 Gewebekulturschalen, die jeweils 96 Vertiefungen enthielten, verteilt und ohne Wechsel des Mediums bei 37°C in einer Atmosphäre von 5% CO₂ und einer relativen Luftfeuchtigkeit von 98% 11 Tage lang inkubiert.

Die Antikörper zeichnen sich aus durch ihre inhibierende Wirkung auf die TNF-Bindung an HL60-Zellen oder durch ihre Bindung an Antigen im Filtertest gemäss Beispiel 1. Zum Nachweis der biologischen Aktivität von anti(TNF-BP)-Antikörpern wurde folgendermassen verfahren: 5x10⁶ HL60 oder U937-Zellen wurden in vollständigem RPMI 1640 Medium zusammen mit affinitätsgereinigten monoklonalen anti--(TNF-BP)-Antikörpern oder Kontrollantikörpern (d.h. solchen, die nicht gegen TNF-BP gerichtet sind) in einem Konzentrationsbereich von 1 ng/ml bis 10 µg/ml inkubiert. Nach einer Stunde Inkubation bei 37°C wurden die Zellen durch Zentrifugation gesammelt und mit 4,5 ml PBS bei 0°C gewaschen. Sie wurden in 1 ml vollständigem RPMI 1640 Medium (Beispiel 2), das zusätzlich 0,1% Natriumazid und ¹²⁵I--TNFα (10⁶ cpm/ml) mit oder ohne Beigabe von unmarkiertemTNFa (s.o.)enthielt, resuspendiert. Die spezifische Radioaktivität des ¹²⁵I-TNFα betrug 700 Ci/mmol.
Die Zellen wurden 2 Stunden bei 4°C inkubiert, gesammelt und 4 mal mit 4,5 ml PBS, das 1% BSA und 0,001% Triton X 100 (Fluka) enthielt, bei 0°C gewaschen. Die an die Zellen gebundene Radioaktivität wurde in einem γ-Scintillationszähler gemessen. In einem vergleichbaren Experiment wurde die zellgebundene Radioaktivität von Zellen, die nicht mit anti-(TNF-BP)-Antikörpern behandelt worden waren, bestimmt (ungefähr 10 000 cpm/5x10⁶ Zellen).

### Beispiel 4

### Affinitätschromatographie

Für die weitere Reinigung wurden jeweils ein gemäss Beispiel 3 erhaltener monoklonaler anti-(55 kD TNF-BP)-Antikörper (2,8 mg/ml Gel), TNFα (3,0 mg/ml Gel) und Rinderserumalbumin (BSA, 8,5 mg/ml Gel) gemäss den Vorschriften des Herstellers kovalent an CNBr-aktivierte Sepharose 4B (Pharmacia, Uppsala, Schweden) gekoppelt. Der gemäss Beispiel 2 erhaltene Zellextrakt wurde über die so hergestellten und in der folgenden Reihenfolge hintereinandergeschalteten Säulen geleitet: BSA-Sepharose-Vocsäule, Immunaffinitätssäule [Anti-(55 kD-TNF-BP)-Antikörper], TNFα-Ligand-Affinitätssäule. Nach vollständigem Auftrag wurden die beiden letztgenannten Säulen abgetrennt und einzeln für sich mit je 100 ml der folgenden Pufferlösungen gewaschen: (1) PBS, 1.0% Triton X-100, 10 mM Benzamidin, 100 E/ml Aprotinin; (2) PBS, 0,1% Triton X-100, 0,5M NaCl, 10 mM ATP, 10 mM Benzamidin, 100 E/ml Aprotinin; und (3) PBS, 0,1% Triton X-100, 10 mM Benzamidin, 100 E/ml Aprotinin. Sowohl die Immun- als auch die TNFα-Ligand-Affinitätssäule wurden dann mit 100 mM Glycin pH 2.5, 100 mM NaCl, 0,2% Decylmaltoside, 10 mM Benzamidin, 100 E/ml Aprotinin jede für sich eluiert. Die im Filtertest gemäss Beispiel 1 aktiven Fraktionen jeder Säule wurden danach jeweils vereint und mit 1M Tris pH 8,0 neutralisiert.

Die so vereinten TNF-BP-aktiven Fraktionen der Immun-Affinitätschromatographie einerseits und der TNFα-Ligand-Affinitätschromatographie andererseits wurden zur weiteren Reinigung nochmals auf je eine kleine TNFα-Ligand-Affinitätssäule aufgetragen. Danach wurden diese beiden Säulen mit je 40 ml von (1) PBS, 1,0% Triton X-100, 10 mM Benzamidin, 100 E/ml Aprotinin, (2) PBS, 0,1% Triton X-100, 0,5M NaCl, 10 mM ATP, 10mM Benzamidin, 100 E/ml Aprotinin, (3) PBS, 0.1% Triton X-100, (4) 50 mM Tris pH 7.5, 150 mM NaCl, 1,0% NP-40, 1,0% Desoxycholat, 0,1% SDS, (5) PBS, 0,2% Decylmaltosid gewaschen. Anschliessend wurden die Säulen mit 100 mM Glycin pH 2.5, 100 mM NaCl, 0,2% Decylmaltosid eluiert. Fraktionen von 0,5 ml von jeder Säule wurden für sich gesammelt und die gemäss Filtertest (Beispiel 1) aktiven Fraktionen von jeder Säule jeweils für sich vereint und in einer Centricon-Einheit (Amicon, Molekulargewichts--Ausschluss 10'000) aufkonzentriert.

### Beispiel 5

### Auftrennung mittels HPLC

Die gemäss Beispiel 4 erhaltenen aktiven Fraktionen wurden gemäss ihrer unterschiedlichen Herkunft (Immun- bzw. Ligand-Affinitätschromatographie) jeweils für sich auf C1/C8 Umkehrphasen-HPLC-Säulen (ProRPC, Pharmacia, 5x20 mm), die mit 0,1% Trifluoressigsäure, 0,1% Octylglucosid equilibriert worden waren, aufgetragen. Die Säulen wurden dann mit einem linearen Acetonitril-Gradienten (0-80%) im gleichen Puffer bei einem Fluss von 0.5 ml/min eluiert. Fraktionen von 1,0 ml wurden von jeder Säule gesammelt und die aktiven Fraktionen von jeder Säule für sich vereint (Nachweis gemäss Beispiel 1).

### Beispiel 6

### Auftrennung mittels SDS-PAGE

Die gemäss Beispiel 5 erhaltenen und gemäss Filtertest (Beispiel 1) aktiven Fraktionen wurden durch SDS-PAGE gemäss [34] weiter aufgetrennt. Dazu wurden die Proben in SDS-Probenpuffer während 3 Minuten auf 95°C erhitzt und anschliessend auf einem 12% Acrylamid-Trenngel mit einem 5%igen Sammelgel elektrophoretisch aufgetrennt. Als Referenz zur Bestimmung der scheinbaren Molekulargewichte auf dem SDS-PAGE Gel wurden die folgenden Eichproteine verwendet: Phosphorylase B (97,4 kD), BSA (66,2 kD), Ovalbumin (42,7 kD), Carboanhydrase (31,0 kD), Soya Trypsin-Inhibitor (21,5 kD) und Lysozym (14,4 kD).

Unter den genannten Bedingungen wurden für Proben, die gemäss Beispiel 4 durch TNF-α-Ligandenaffinitätschromatographie von Immunaffinitätschromatographieeluaten erhalten und durch HPLC gemäss Beispiel 5 weiter aufgetrennt worden waren, zwei Banden von 55 kD und 51 kD sowie drei schwächere Banden von 38 kD, 36 kD und 34 kD erhalten. Diese Banden wurden in einem Mini Trans Blot System (BioRad, Richmond, California, USA) elektrophoretisch während 1 Stunde bei 100 V in 25 mM Tris, 192 mM Glycin, 20% Methanol auf eine PVDF-Membran (Immobilon, Millipore, Bedford, Mass. USA) transferiert. Danach wurde die PVDF-Membran entweder mit 0,15% Serva-Blau (Serva, Heidelberg, BRD) in Methanol/Wasser/Eisessig (50/40/10 Volumenteile) auf Protein gefärbt oder mit entfettetem Milchpulver blockiert und anschliessend zum Nachweis von Banden mit TNF-BP-Aktivität mit ¹²⁵I--TNFα gemäss den in Beispiel 1 beschriebenen Filtertestbedingungen inkubiert. Dabei zeigte sich, dass alle in der Proteinfärbung zur Darstellung gelangten Banden spezifisch TNFα banden. Alle diese Banden banden im Western Blot nach Towbin et al. [38] auch den gemäss Beispiel 3 hergestellten monoklonalen Anti-55kD-TNF-BP-Antikörper. Dabei wurde ein gemäss dem in Beispiel 1 beschriebenen Verfahren mit Na¹²⁵ I radioaktiv markierter, affinitätsgereinigter (Mausimmunglobulin-Sepharose-4B-Affinitätssäule) Kaninchen-anti-Maus-Immunoglobulin-Antikörper zum autoradiographischen Nachweis dieses Antikörpers eingesetzt.

Proben, die gemäss Beispiel 4 durch zweimalige TNF-α--Ligandenaffinitätschromatographie des Durchlaufs der Immunaffinitätschromatographie erhalten und durch HPLC gemäss Beispiel 5 weiter aufgetrennt worden waren, zeigten unter den oben spezifizierten SDS-PAGE- und Blottransfer-Bedingungen zwei zusätzliche Banden von 75 kD und 65 kD, die beide im Filtertest (Beispiel 1) spezifisch TNF banden. Im Western Blot gemäss Towbin et al. (s.o.) reagierten die Proteine dieser beiden Banden nicht mit dem gemäss Beispiel 3 hergestellten anti-(55 kD TNF-BP)-Antikörper. Sie reagierten allerdings mit einem monoklonalen Antikörper, der ausgehend von der 75 kD-Bande (anti-75 kD TNF-BP-Antikörper) gemäss Beispiel 3 erzeugt worden war.

### Beispiel 7

### Aminosäuresequenzanalyse

Zur Aminosäuresequenzanalyse wurden die gemäss Beispiel 5 erhaltenen und gemäss Filtertest (Beispiel 1) aktiven Fraktionen mittels der in Beispiel 6 beschriebenen, nun jedoch reduzierenden, SDS-PAGE Bedingungen (SDS-Probenpuffer mit 125 mM Dithiothreitol) aufgetrennt. Es wurden die gleichen Banden wie gemäss Beispiel 6 gefunden, die allerdings auf Grund der reduzierenden Bedingungen der SDS-PAGE im Vergleich zu Beispiel 6 alle um etwa 1-2 kD höhere Molekulargewichte zeigten. Diese Banden wurden dann gemäss Beispiel 6 auf PVDF-Membranen übertragen und mit 0.15% Serva-Blau in Methanol/Wasser/Eisessig (50/40/10 Volumenteile) während 1 Minute gefärbt, mit Methanol/Wasser/Eisessig (45/48/7 Volumenteile) entfärbt, mit Wasser gespült, luftgetrocknet und danach ausgeschnitten. Bei sämtlichen Schritten wurden zur Vermeidung von N-terminaler Blockierung die von Hunkapiller [34] angegebenen Bedingungen eingehalten. Zunächst wurden die gereinigten TNF-BP unverändert zur Aminosäuresequenzierung eingesetzt. Um zusätzliche Sequenzinformation zu erhalten, wurden die TNF-BP nach Reduktion und S-Carboxymethylierung [Jones, B.N. (1986) in "Methods of Protein Microcharacterisation", J.E. Shively, ed., Humana Press, Clifton NJ, 124-125] mit Bromcyan (Tarr, G.E. in "Methods of Protein Microcharacterisation", 165-166, op.cit.), Trypsin und/oder Proteinase K gespalten und die Peptide mittels HPLC nach bekannten Methoden der Proteinchemie aufgetrennt. So vorbereitete Proben wurden dann in einem automatisierten Gasphasen-Mikrosequenzier-Gerät (Applied Biosystems Modell 470A, ABI, Foster City, Calif., USA) mit einem on-line nachgeschalteten automatisierten HPLC PTH-Aminosäureanalysator (Applied Biosystems Modell 120, ABI s.o.) sequenziert, wobei die folgenden Aminosäuresequenzen bestimmt wurden:
1., Für die 55 kD-Bande (gemäss nichtreduzierender SDS-PAGE): 
Leu-Val-Pro-His-Leu-Gly-Asp-Arg-Glu-Lys-Arg-Asp-Ser-Val-
Cys-Pro-Gln-Gly-Lys-Tyr-Ile-His-Pro-Gln-X-Asn-Ser-Ile,
 und 
Ser-Thr-Pro-Glu-Lys-Glu-Gly-Glu-Leu-Glu-Gly-Thr-Thr-Thr-
Lys
 wobei X für einen Aminosäurerest steht, der nicht bestimmt werden konnte.
2., Für die 51 kD und die 38 kD-Banden (gemäss nichtreduzierender SDS-PAGE): Leu-Val-Pro-His-Leu-Gly-Asp-Arg-Glu
3., Für die 65 kD-Bande (gemäss nichtreduzierender SDS-PAGE):
   Bei der N-terminalen Sequenzierung der 65 kD Bande wurden bis zum 15. Rest ohne Unterbrechung zwei parallele Sequenzen ermittelt. Da eine der beiden Sequenzen einer Teilsequenz des Ubiquitins [36,37] entsprach, wurde für die 65 kD-Bande die folgende Sequenz abgeleitet: 
Leu-Pro-Ala-Gln-Val-Ala-Phe-X-Pro-Tyr-Ala-Pro-Glu-Pro-Gly-
Ser-Thr-Cys.
 wobei X für einen Aminosäurerest steht, der nicht bestimmt werden konnte.

Weitere Peptidsequenzen für 75(65)kDa-TNF-BP wurden bestimmt: Ile-X-Pro-Gly-Phe-Gly-Val-Ala-Tyr-Pro-Ala-Leu-Glu und 
Ser-Gln-Leu-Glu-Thr-Pro-Glu-Thr-Leu-Leu-Gly-Ser-Thr-Glu-
Glu-Lys-Pro-Leu
 und Val-Phe-Cys-Thr und 
Asn-Gln-Pro-Gln-Ala-Pro-Gly-Val-Glu-Ala-Ser-Gly-Ala-Gly-
Glu-Ala
 und Leu-Cys-Ala-Pro und Val-Pro-His-Leu-Pro-Ala-Asp und Gly-Ser-Gln-Gly-Pro-Glu-Gln-Gln-X-X-Leu-Ile-X-Ala-Pro wobei X für einen Aminosäurerest steht, der nicht bestimmt werden konnte.

### Beispiel 8

### Bestimmung von Basen-Sequenzen von komplementärer DNA (cDNA)

Ausgehend von der Aminosäuresequenz gemäss Formel IA wurden unter Berücksichtigung des genetischen Codes zu den Aminosäureresten 2-7 und 17-23 entsprechende, vollständig degenerierte Oligonucleotide in geeigneter Komplementarität synthetisiert ("sense" and "antisense" Oligonucleotide). Totale zelluläre RNA wurde aus HL60-Zellen isoliert [42, 43], und der erste cDNA-Strang durch Oligo-dT-Priming oder durch Priming mit dem "antisense" Oligonucleotid mittels eines cDNA-Synthese-Kits (RPN 1256, Amersham, Amersham, England) gemäss der Anleitung des Herstellers synthetisiert. Dieser cDNA-Strang und die beiden synthetisierten degenerierten "sense" und "anti-sense" Oligonucleotide wurden in einer Polymerase-Kettenreaktion (PCR, Perkin Elmer Cetus, Norwalk, CT, USA gemäss Anleitung des Herstellers) dazu verwendet, die für die Aminosäure-Reste 8-16 (Formel IA) codierende Basesequenz als cDNA-Fragment zu synthetisieren. Die Basensequenz dieses cDNA-Fragmentes lautet: 5'-AGGGAGAAGAGAGATAGTGTGTGTCCC-3'. Dieses cDNA-Fragment wurde als Probe verwendet, um nach bekannten Verfahren einen für das 55 kD TNF-BP codierenden cDNA-Klon in einer λgt11-cDNA-Genbank von menschlicher Placenta zu identifizieren (42,43). Dieser Klon wurde dann nach üblichen Methoden aus dem λ-Vektor geschnitten und in die Plasmide pUC18 (Pharmacia, Uppsala, Sweden) und pUC19 (Pharmacia, Uppsala, Sweden) und in die M13mp18/M13mp19 Bacteriophagen (Pharmacia, Uppsala, Sweden) kloniert (42,43). Die Nukleotidsequenz dieses cDNA-Klons wurde mit einem Sequenase-Kit (U.S. Biochemical, Cleveland, Ohio, USA) nach den Angaben des Herstellers bestimmt. Die Nukleotidsequenz und die daraus abgeleitete Aminosäuresequenz für das 55 kD TNF-BP und dessen Signalpeptid (Aminosäure "-28" bis Aminosäure "0") ist in Figur 1 mittels der im Stand der Technik üblichen Abkürzungen für Basen wie Aminosäuren dargestellt. Aus Sequenzvergleichen mit anderen, bereits bekannten Rezeptorproteinsequenzen lassen sich ungefähr 180 Aminosäuren enthaltende N-terminale wie 220 Aminosäure enthaltende C-terminale Domänen, die von einer nach den Sequenzvergleichen typischen Transmembran-Region von 19 Aminosäuren (in Figur 1 unterstrichen) getrennt werden, bestimmen. Hypothetische Glykosylierungsstellen sind in Figur 1 durch Sterne über der entsprechenden Aminosäure gekennzeichnet.

Im Wesentlichen analoge Techniken wurden dazu eingesetzt, 75/65 kD TNF-BP codierende partielle cDNA-Sequenzen zu identifizieren, wobei allerdings in diesem Fall genomische humane DNA und von Peptid IIA abgeleitete, vollständig degenerierte 14-mere und 15-mere "sense" und "antisense" Oligonucleotide verwendet wurden, um eine primäre, 26 bp cDNA-Probe in einer Polymerase-Kettenreaktion herzustellen. Diese cDNA-Probe wurde dann dazu verwendet, in einer HL-60 cDNA-Bibliothek cDNA-Klone von verschiedener Länge zu identifizieren. Diese cDNA-Bibliothek wurde mittels isolierter HL60 RNA und einem cDNA-Klonierungskit (Amersham) nach den Angaben des Herstellers hergestellt. Die Sequenz eines solchen cDNA-Klons ist in Figur 4 dargestellt, wobei nochmalige Sequenzierung zu folgender Korrektur führte. An Stelle des Serins in Position 3 muss ein Threonin das von "ACC" nicht von "TCC" kodiert wird, stehen.

### Beispiel 9

### Expression in COS 1-Zellen

Für die Expression in COS-Zellen wurden Vektoren ausgehend von dem Plasmid "pN11" konstruiert. Das Plasmid "pN11" enthält den effizienten Promotor und Enhancer des "major immediate-early" Gens des menschlichen Cytomegalo-virus ("HCMV"; Boshart et al., Cell 41, 521-530, 1985). Hinter dem Promotor befindet sich eine kurze DNA-Sequenz, welche mehrere Restriktionsschnittstellen enthält, die nur einmal im Plasmid vorkommen ("Polylinker"), u.a. die Schnittstellen für HindIII, BalI, BamHI und PvuII (siehe Sequenz). 
PvuII
5'-AAGCTTGGCCAGGATCCAGCTGACTGACTGATCGCGAGATC-3'
3'-TTCGAACCGGTCCTAGGTCGACTGACTGACTAGCGCTCTAG-5'
 Hinter diesen Schnittstellen befinden sich drei Translations-Stopcodons in allen drei Leserastern. Hinter der Polylinkersequenz befindet sich das 2. Intron und das Polyadenylierungssignal des Präproinsulingens der Ratte (Lomedico et al., Cell 18, 545-558, 1979). Das Plasmid enthält ferner den Replikationsursprung des SV40 Virus sowie ein Fragment aus pBR322, das E. coli-Bakterien Ampicillin-Resistenz verleiht und die Replikation des Plasmids in E. coli ermöglicht.

Zur Konstruktion des Expressionsvektors "pN123" wurde dieses Plasmid "pN11" mit der Restriktionsendonuklease PvuII geschnitten und anschliessend mit alkalischer Phosphatase behandelt. Der dephosphorylierte Vektor wurde danach aus einem Agarosegel isoliert (V1). Die 5'-überhängenden Nukleotide des EcoRI-geschnittenen 1,3kb-Fragments der 55 kD TNF-BP-cDNA (siehe Beispiel 8) wurden mit Hilfe von Klenow-Enzym aufgefüllt. Anschliessend wurde dieses Fragment aus einem Agarosegel isoliert (F1). Danach wurden V1 und F1 mittels T4-Ligase miteinander verbunden. E. coli HB101-Zellen wurden dann mit diesem Ligierungsansatz nach bekannten Methoden [42] transformiert. Mit Hilfe von Restriktionsanalysen und DNA-Sequenzierung nach bekannten Methoden [42] wurden Transformanten identifiziert, die mit einem Plasmid transformiert worden waren, welches das 1.3kb EcoRI-Fragment der 55 kD TNF-BP-cDNA in der für die Expression über den HCMV-Promotor korrekten Orientierung enthielt. Dieser Vektor erhielt die Bezeichnung "pN123".

Zur Konstruktion des Vektors "pK19" wurde folgendermassen verfahren. Ein DNA-Fragment, welches nur die für den extrazellulären Teil des 55 kD TNF-BP codierende cDNA enthält (Aminosäuren -28 bis 182 gemäss Figur 1) wurde mittels PCR-Technologie erhalten (Saiki et al., Science 230, 1350-1354, 1985, siehe auch Beispiel 8). Die folgenden Oligonukleotide wurden, um die für den extrazellulären Teil des 55 kD TNF-BP codierende cDNA aus "pN123" zu amplifizieren, verwendet:

BAMHI
5'-CACAGGGATCCATAGCTGTCTGGCATGGGCCTCTCCAC-3'
ASP718
3'-CGTGACTCCTGAGTCCGTGGTGTATTATCTCTAGACCATGGCCC-5'

Durch diese Oligonukleotide wurden ebenfalls zwei Stopkodons der Translation hinter Aminosäure 182 eingeführt. Das so amplifizierte DNA-Fragment wurde mit BamHI und Asp718 geschnitten, die hierbei entstandenen überstehenden Enden mit Hilfe des Klenow-Enzyms aufgefüllt und dieses Fragment anschliessend aus einem Agarosegel isoliert (F2). F2 wurde dann mit V1 ligiert und der gesamte Ansatz zur Transformation von E. coli HB101, wie bereits beschrieben, verwendet. Transformanten, die mit einem Plasmid transformiert worden waren, welches das DNA-Fragment in der für die Expression über den HCMV-Promotor korrekten Orientierung enthielten, wurden mittels DNA-Sequenzierung (s.o.) identifiziert. Das daraus isolierte Plasmid erhielt die Bezeichnung "pK19".

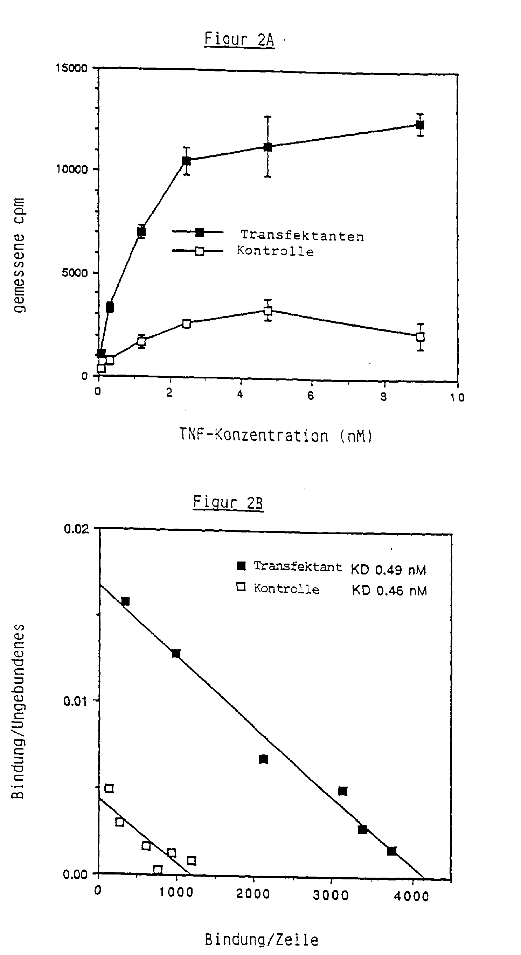

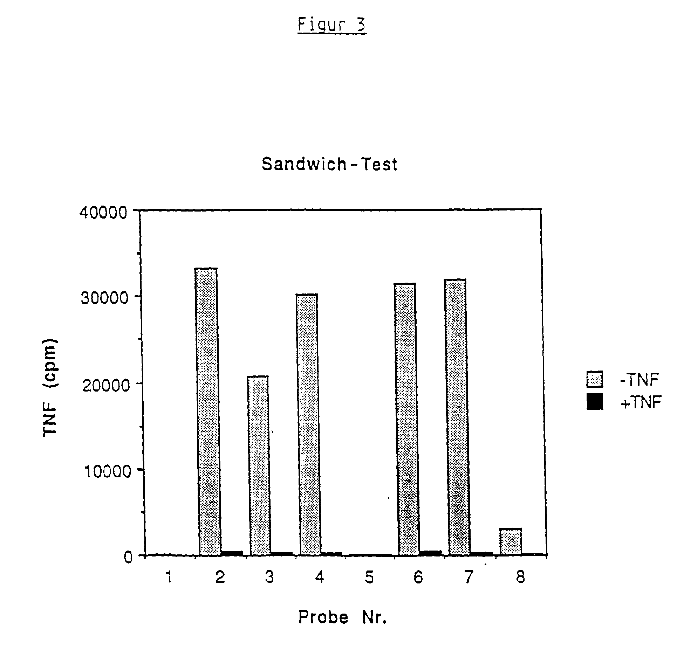

Transfektion der COS-Zellen mit den Plasmiden "pN123" oder "pK19" wurde nach der von Felgner et al. veröffentlichten Lipofections-Methode (Proc. Natl. Acad. Sci. USA 84, 7413-7417, 1987) durchgeführt. 72 Stunden nach erfolgter Transfektion wurden die mit "pN123" transfizierten Zellen nach bekannten Methoden mit ¹²⁵I-TNFα auf Bindung analysiert. Das Resultat der Scatchard-Analyse [Scatchard, G., Ann. N.Y. Acad. Sci. 51, 660, 1949] der so erhaltenen Bindungsdaten (Figur 2A) ist in Figur 2B dargestellt. Die Kulturüberstände der mit "pK19" transfizierten Zellen wurden in einem "Sandwich"-Test untersucht. Dazu wurden PVC-Microtiterplatten (Dynatech, Arlington, VA, USA) mit 100 µl/Loch eines Kaninchen-anti-Maus Immunglobulins (10 µg/ml PBS) sensibilisiert. Anschliessend wurde die Platte gewaschen und mit einem anti-55 kD TNF-BP-Antikörper, der gemäss Beispiel 3 durch seine Antigenbindung nachgewiesen und isoliert wurde, der aber die TNF-Bindung an Zellen nicht inhibiert, inkubiert (3 Stunden, 20°C). Die Platte wurde dann wieder gewaschen und über Nacht bei 4°C mit 100 µl/Loch der Kulturüberstände (1:4 verdünnt mit 1% entfetteter Milchpulver enthaltendem Puffer A: 50 mM Tris/HCl pH 7.4, 140 mM NaCl, 5 mM EDTA, 0,02% Na-Azid) inkubiert. Die Platte wurde entleert und mit ¹²⁵I-TNFα enthaltendem Puffer A (10⁶ cpm/ml, 100 µl/Loch) mit oder ohne Zusatz von 2 µg/ml unmarkiertem TNF während 2 Stunden bei 4°C inkubiert. Danach wurde die Platte 4 mal mit PBS gewaschen, die einzelnen Löcher wurden ausgeschnitten und in einem γ-Zähler gemessen. Die Resultate von 5 parallelen Transfektionen (Säulen # 2, 3, 4, 6 und 7), von zwei Kontroll-Transfektionen mit dem pN11-Vektor (Säulen # 1, 5) und von einer Kontrolle mit HL60-Zell-Lysat (Säule # 8) sind in Figur 3 dargestellt.

### Beispiel 10

### Expression in Insektenzellen

Für die Expression in einem Baculovirus-Expressions-system wurde von dem Plasmid "pVL941" (Luckow und Summers, 1989, "High Level Expression of Nonfused Foreign Genes with Autographa california Nuclear Polyhedrosis virus Expression Vectors", Virology 170, 31-39) ausgegangen und dieses folgendermassen modifiziert. Es wurde die einzige EcoRI--Restriktionsschnittstelle in "pVL941" entfernt, indem das Plasmid mit EcoRI geschnitten und die überstehenden 5'-Enden mit Klenow-Enzym aufgefüllt wurden. Das hieraus erhaltene Plasmid pVL941/E- wurde mit BamHI und Asp718 verdaut und der Vektorrumpf anschliessend aus einem Agarosegel isoliert. Dieses Fragment wurde mit einem synthetischen Oligonukleotid der folgenden Sequenz ligiert:

BamHI   EcoRI        Asp718
5' - GATCCAGAATTCATAATAG      - 3'
3' -     GTCTTAAGTATTATCCATG - 5'

E. coli HB101 wurde mit dem Ligierungsansatz transformiert und Transformanten, die ein Plasmid enthielten, in welches das Oligonukleotid korrekt eingebaut worden war, wurden durch Restriktionsanalyse und DNA-Sequenzierung nach bekannten Methoden (s.o.) identifiziert; dieses Plasmid wurde "pNR704" genannt. Zur Konstruktion des Transfervektors "pN113" wurde dieses Plasmid "pNR704" mit EcoRI geschnitten, mit alkalischer Phosphatase behandelt und der so erzeugte Vektorrumpf (V2) anschliessend aus einem Agarosegel isoliert. Das wie oben mit EcoRI geschnittene 1,3 kb-Fragment der 55 kD TNF-BP-cDNA wurde mit Fragment V2 ligiert. Mit diesem Ligierungsansatz erhaltene Transformanten, die ein Plasmid enthielten, welches das cDNA-Insert in der korrekten Orientierung für die Expression über den Polyhedrinpromotor enthielten, wurden identifiziert (s.o.). Der daraus isolierte Vektor erhielt die Bezeichnung "pN113".

Zur Konstruktion des Transfervektors "pN119" wurde folgendermassen vorgegangen. Das 1,3 kb EcoRI/EcoRI-Fragment der 55 kD TNF-BP cDNA in dem "pUC19"-Plasmid (siehe Beispiel 8) wurde mit BanI verdaut und mit dem folgenden synthetischen Oligonukleotid ligiert:

BanI                    Asp718
5' - GCACCACATAATAGAGATCTGGTACCGGGAA - 3'
3' -     GTGTATTATCTCTAGACCATGGCCC     - 5'

Mit dem obigen Adaptor werden zwei Stopcodons der Translation hinter Aminosäure 182 und eine Schnittstelle für die Restriktionsendonuklease Asp718 eingebaut. Nach erfolgter Ligation wurde der Ansatz mit EcoRI und Asp718 verdaut und das partielle 55 kD TNF-BP-Fragment (F3) isoliert. Weiterhin wurde das ebenfalls mit Asp718 und EcoRI geschnittene Plasmid "pNR704" mit F3 ligiert und der Ligierungsansatz in E. coli HB101 transformiert. Die Identifikation der Transformanten, welche ein Plasmid enthielten, in das die partielle 55 kD TNF-BP cDNA korrekt für die Expression integriert worden war, erfolgte wie bereits beschrieben. Das aus diesen Transformanten isolierte Plasmid erhielt den Namen "pN119".

Zur Konstruktion des Transfervektors "pN124" wurde folgendermassen vorgegangen. Das in Beispiel 9 beschriebene, für den extrazellulären Teil des 55 kD TNF-BP codierende cDNA-Fragment wurde mit den angegebenen Oligonukleotiden mit Hilfe der PCR-Technologie, wie in Beispiel 9 beschrieben, amplifiziert. Dieses Fragment wurde mit BamHI und Asp718 geschnitten und aus einem Agarosegel isoliert (F4). Das Plasmid "pNR704" wurde ebenfalls mit BamHI und Asp718 geschnitten und der Vektorrumpf (V4) wurde isoliert (s.o.). Die Fragmente V4 und F4 wurden ligiert, E. coli HB101 damit transformiert und der rekombinante Transfervektor "pN124" wurde, wie beschrieben, identifiziert und isoliert.

Zur Transfektion der Insektenzellen wurde folgendermassen vorgegangen. 3 µg des Transfervektors "pN113" wurden mit 1 µg DNA des Autographa californica-Nuklearpolyhedrosisvirus (AcMNPV) (EP 127839) in Sf9-Zellen (ATCC CRL 1711) transfektiert. Polyhedrin negative Viren wurden identifiziert und aus "Plaques" gereinigt [52]. Mit diesen rekombinanten Viren wurden wiederum Sf9 Zellen wie in [52] beschrieben, infiziert. Nach 3 Tagen in Kultur wurden die infizierten Zellen auf Bindung von TNF mittels ¹²⁵I-TNFα untersucht. Dazu wurden die transfektierten Zellen mit einer Pasteurpipette von der Zellkulturschale abgewaschen und bei einer Zelldichte von 5x10⁶ Zellen/ml Kulturmedium [52], das 10 ng/ml ¹²⁵I-TNF-α enthielt, sowohl in Anwesenheit wie Abwesenheit von 5 µg/ml nichtmarkiertem TNF-α resuspendiert und 2 Stunden auf Eis inkubiert. Danach wurden die Zellen mit reinem Kulturmedium gewaschen und die zellgebundene Radioaktivität in einem γ-Zähler gezählt (siehe Tabelle 2).

**Tabelle 2**

| Zellen | Zellgebundene Radioaktivität pro 10⁶ Zellen |
|---|---|
| nichtinfizierte Zellen (Kontrolle) | 60 cpm |
| infizierte Zellen | 1600 ± 330 cpm ¹⁾ |

| | |
|---|---|
| ¹⁾ Mittelwert und Standardabweichung aus 4 Experimenten | |

### Beispiel 11

Analog zu dem in Beispiel 9 beschriebenen Verfahren wurde das für den extrazellulären Bereich des 55 kDa TNF-BP codierende cDNA-Fragment, nun jedoch mit den folgenden Oligonukleotiden als Primer, in einer Polymerasen-Kettenreaktion amplifiziert:
Oligonukleotid 1: 
Sst I
5'-TAC GAG CTC GGC CAT AGC TGT CTG GCA TG-3'
Oligonukleotid 2:

Sst I
5'-ATA GAG CTC TGT GGT GCC TGA GTC CTC AG-3'

Dieses cDNA-Fragment wurde in den pCD4-Hγ3-Vektor [DSM 5523; Europäische Patentanmeldung Nr. 90107393.2; Japanische Patentanmeldung Nr. 108967/90; US Patent Application Ser.No. 510773/90] ligiert, aus dem die CD4-cDNA über die Sst I-Restriktions-Schnittstellen herausgenommen worden war. SstI-Schnittstellen befinden sich in dem Vektor pCD4-Hγ3 sowohl vor wie in dem CD4-Teilsequenzstück wie dahinter. Das Konstrukt wurde mittels Protoplastenfusion nach Oi et al. (Procd. Natl. Acad. Sci. USA 80, 825-829, 1983) in J558-Myelomzellen (ATCC Nr. TIB6) transfiziert. Transfektanten wurden durch Zugabe von 5 µg/ml Mycophenolsäure und 250 µg/ml Xanthin (Traunecker et al., Eur. J. Immunol. 16, 851-854 [1986]) in das Grundmedium (Dulbecco's modifiziertes Eagle's Medium, 10% fötales Kälberserum, 5 x 10⁻⁵M 2-Mercaptoethanol) selektioniert. Das von den transfizierten Zellen sekretierte Expressionsprodukt konnte mittels üblicher Methoden der Proteinchemie, z.B. TNF-BP-Antikörper--Affinitätschromatographie, gereinigt werden. Falls nicht bereits spezifisch angegeben, wurden zur Kultivierung der verwendeten Zelllinien, zum Klonieren, Selektionieren bzw. zur Expansion der klonierten Zellen Standardverfahren, wie z.B. von Freshney, R.I. in "Culture of Animal Cells", Alan R. Liss, Inc., New York (1983) beschrieben, verwendet.
- 1.: G.E. Nedwin, S.L. Naylor, A.Y. Sakaguchi, D. Smith, J. Jarrett-Nedwin, D. Pennica, D.V. Goeddel and P.W. Gray: Nucl. Acids Res. 13, 6361, 1985
- 2.: B. Beutler and A. Cerami: New England J. Med. 316, 379, 1987
- 3.: L.J. Old: Science 230, 630, 1985
- 4.: G. Trinchieri, M. Kobayashi, M. Rosen, R. Loudon, M. Murphy and B. Perussia: J. exp. Med. 164, 1206, 1986
- 5.: J. Vilcek, V.J. Palombella, D. Henriksen-de Stefano, C. Swenson, R. Feinman, M. Hirai and M. Tsujimoto: J. exp. Med. 163, 632, 1986
- 6.: B.J. Sugarman, B.B. Aggarwal, P.E. Hass, I.S. Figari, M.A. Palladino and H.M. Shepard: Science 230, 943, 1985
- 7.: J.R. Gamble, J.M. Harlan, S.J. Klebanoff and M.A. Vadas: Proc. Natl. Acad. Sci. USA 82, 8667, 1985
- 8.: N. Sato, T. Goto, K. Haranaka, N. Satomi, H. Nariuchi, Y. Mano and Y. Sawasaki: J. Natl. Cancer Inst. 76, 1113, 1986
- 9.: A.H. Stolpen, E.C. Guinan, W. Fiers and J.S. Pober: Am. J. Pathol. 123, 16, 1986
- 10.: J.S. Pober, L.A. Lapierre, A.H. Stolpen, T.A. Brock, T.A. Springer, W. Fiers, M.P. Bevilacqua, D.L. Mendrick and M.A Gimbrone: J. Immunol. 138, 3319, 1987
- 11.: M. Kawakami, P. Pekala, M. Lane and A. Cerami: Proc. Natl. Acad. Sci. USA 79, 912, 1982
- 12.: T. Collins, L.A. Lapierre, W. Fiers, J.L. Strominger and J.S Pober: Proc. Natl. Acad. Sci. USA 83, 446, 1986
- 13.: G.H.W. Wong and D.V. Goeddel: Nature 323, 819, 1986
- 14.: J.W. Lowenthal, D.W.Ballard, E. Böhnlein and W.C. Greene: Proc. Natl. Acad. Sci. USA 86, 2331, 1989
- 15.: M.J. Lenardo, C.M. Fan, T. Maniatis and D. Baltimore: Cell 57, 287, 1989
- 16.: A.E. Goldfeld and T. Maniatis: Proc. Natl. Acad. Sci. USA 86, 1490, 1989
- 17.: A. Waage, A. Halsteuren and T. Espevik: Lancet, Febr. 14, 1987, 355,
- 18.: C.O. Jacob and H.O. McDevitt: Nature 331, 356, 1988
- 19.: G.E. Grau, L.F. Fajardo, P. Piguet. B. Allet, P. Lambert and P. Vassalli: Science 237, 1210, 1987
- 20.: B. Beutler, I.W. Milsark and A.C. Cerami: Science 229, 869, 1985
- 21.: B.B. Aggarwal, T.E. Eessalu and P.E. Hass: Nature 318, 665, 1985
- 22.: M. Tsujimoto, Y.K. Yip and J. Vilcek: Proc. Natl. Acad. Sci. USA 82, 7626, 1985
- 23.: C. Baglioni, S. McCandless, J. Tavernier and W. Fiers: J. Biol. Chem. 260, 13395, 1985
- 24.: P. Hohmann, R. Remy, M. Brockhaus and A.P.G.M. van Loon: J. Biol. Chem., im Druck
- 25.: F.C. Kull, S. Jacobs and P. Cuatrecasas: Proc. Natl. Acad. Sci. USA 82, 5756, 1985
- 26.: A.A. Creasy, R. Yamamoto and Ch.R. Vitt: Proc. Natl. Acad. Sci. USA 84, 3293, 1987
- 27.: G.B. Stauber, R.A. Aiyer and B.B. Aggarwal: J. Biol. Chem. 263, 19098, 1988
- 28.: K. Hirano, K. Yamamoto, Y. Kobayashi and T. Osawa: J. Biochem. 105, 120, 1989
- 29.: Y. Niitsu, N. Watanabe, H. Sone, H. Neda, N. Yamauchi, M. Maeda and I. Urushizaki: J. Biol. Resp. Modifiers 7, 276, 1988
- 30.: I. Olsson, A. Grubb, U. Gullberg, M. Lantz, E. Nilsson, C. Peetre and H. Thysell: Abstract, 2nd Intern. Conference on Tumor Necrosis Factor and Related Cytokines, Napa, California, 15.-20. Januar 1989
- 31.: H.R. Lötscher and M. Brockhaus: Abstract, 2nd Intern. Conference on Tumor Necrosis Factor and Related Cytokines, Napa, California, 15.-20. Januar 1989
- 32.: M. Brockhaus, H. Lötscher, H.-P. Hohmann und W. Hunziker: Abstract, 2nd Intern. Conference on Tumor Necrosis Factor and Related Cytokines, Napa, California, 15.-20. Januar 1989
- 33.: C.R. Cantor and P.R. Schimmel, in Biophysical Chemistry, W.H. Freeman, ed., San Francisco, 1980, p. 850
- 34.: M.W. Hunkapiller, E. Lujan, F. Ostrander, L.E. Hood: Methods Enzymol. 91, 227, 1983
- 35.: U.K. Lämmli: Nature 227, 680, 1970
- 36.: T.St. John, W.M. Gallatin, M. Siegelman. H.T. Smith, V.A. Fried and I.L. Weissman: Science 231, 845, 1986
- 37.: M. Siegelman, M.W. Bond, W.M. Gallatin, T.St. John, H.T. Smith, V.A. Fried and I.L. Weissman: Science 231, 823, 1986
- 38.: H. Towbin, T. Staehelin and J. Gordon: Proc. Natl. Acad.Sci. USA 76, 4350, 1979
- 39.: Dinarello, Ch.A., in Lymphokines, Vol. 14, E. Pick, ed., p. 1, Academic Press, London, 1987
- 40.: D.J. Merchant, R.H. Kahn and W.H. Murphy: Handbook of Cell and Organ Culture, Burgess Publ. Co., Minneapolis, 1969
- 41.: G.E. Grau, T.E. Taylor, M.E. Molyneux, J.J. Wirima, P. Vassalli, M. Hommel and P. Lambert: New Engl. J. Med. 320, 1586, 1989
- 42.: J. Sambrook, E.F. Fritsch and T. Maniatis: Molecular Cloning, A Laboratory Manual, 2nd ed., Cold Spring Harbor Laboratory, Cold Spring Harbor, Cold Spring Harbor Laboratory Press, 1989
- 43.: F.M. Ausubel, R. Brent, R.E. Kingston, D.D. Moore, J.A. Smith, J.G. Seidman and K. Struhl: Current Protocols in Molecular Biology 1987-1988, S. Wiley and Sons, New York, 1987
- 44.: E. Harlow and D. Lane: Antibodies, A Laboratory Manual, Cold Spring Harbor Laboratory Publications, New York, 1988
- 45.: S.Fazekas de St. Groth and D. Scheidegger: J. Immunol. Methods 35, 1, 1980
- 46.: D. Pennica and D.V. Goeddel, in Lymphokines, Vol. 13, D.R. Webb and D.V. Goeddel, eds. p. 163, Academic Press. London, 1987
- 47.: J. Tavernier, L. Franzen, A. Marmenout, J. van der Heyden, R. Muller, M. Ruysschaert, A. van Vliet, R. Banden and W. Fiers, in Lymphokines, Vol. 13, D.R. Webb and D.V. Goeddel, eds., p. 181, Academic Press, London
- 48.: P.J. Fraker and J.C. Speck: Biochem. Biophys. Res. Commun. 80, 849, 1987
- 49.: D.H. Erlich, D.H. Gelfand, R.K. Saiki: Nature 331, 61, 1988
- 50.: Bosserhoff, J. Wallach and R.W. Frank: J. Chromatogr. 473, 71, 1989
- 51.: R. Lathe: J. Mol. Biol. 183, 1, 1985
- 52.: Luckow and Summers, "A Manual of Methods for Baculovirus Vectors and Insect Cell Culture Procedures", Texas Agricultural Experimental Station, Texas A & M University, Bulletin Nr. 1555, 2nd edition, 1988

## Patentansprüche

1. Nichtlösliche Proteine und lösliche oder nichtlösliche Fragmente davon, die TNF binden, in homogener Form, sowie deren physiologisch verträgliche Salze.

2. Verbindungen gemäss Anspruch 1, die durch Molekulargewichte gemäss SDS-PAGE unter nichtreduzierenden Bedingungen von etwa 55 kD und 75 kD charakterisiert sind.

3. Verbindungen gemäss einem der Ansprüche 1 und 2, die wenigstens eine der folgenden Aminosäuresequenzen enthalten: 
Leu-Val-Pro-His-Leu-Gly-Asp-Arg-Glu-Lys-Arg-Asp-Ser-Val-Cys-
Pro-Gln-Gly-Lys-Tyr-Ile-His-Pro-Gln-X-Asn-Ser-Ile;

Ser-Thr-Pro-Glu-Lys-Glu-Gly-Glu-Leu-Glu-Gly-Thr-Thr-Thr-Lys;

Leu-Pro-Ala-Gln-Val-Ala-Phe-X-Pro-Tyr-Ala-Pro-Glu-Pro-
Gly-Ser-Thr-Cys;

Ile-X-Pro-Gly-Phe-Gly-Val-Ala-Tyr-Pro-Ala-Leu-Glu;

Ser-Gln-Leu-Glu-Thr-Pro-Glu-Thr-Leu-Leu-Gly-Ser-Thr-Glu-
Glu-Lys-Pro-Leu;

Val-Phe-Cys-Thr;

Asn-Gln-Pro-Gln-Ala-Pro-Gly-Val-Glu-Ala-Ser-Gly-Ala-Gly-
Glu-Ala;

Leu-Cys-Ala-Pro;

Val-Pro-His-Leu-Pro-Ala-Asp;

Gly-Ser-Gln-Gly-Pro-Glu-Gln-Gln-X-X-Leu-Ile-X-Ala-Pro
 wobei X für einen nicht bestimmten Aminosäurerest steht.

4. DNA-Sequenzen, die für nichtlösliche Proteine oder lösliche wie nichtlösliche Fragmente davon, die TNF binden, kodieren, wobei solche DNA-Sequenzen aus den folgenden auswählbar sind:
(a) DNA-Sequenzen, wie sie in Figur 1 oder Figur 4 dargestellt sind, wie deren komplementären Stränge, oder solche, die diese Sequenzen umfassen;
(b) DNA-Sequenzen, die mit wie unter (a) definierten Sequenzen oder Fragmenten davon hybridisieren;
(c) DNA-Sequenzen, die auf Grund der Entartung des genetischen Codes nicht mit Sequenzen, wie unter (a) und (b) definiert, hybridisieren, aber die für Polypeptide mit genau gleicher Aminosäuresequenz kodieren.

5. DNA-Sequenzen gemäss Anspruch 4, die eine Kombination aus zwei Teil-DNA-Sequenzen umfassen, wobei die eine Teilsequenz für lösliche Fragmente von nichtlöslichen Proteinen, die TNF binden kodiert und die andere Teil-Sequenz, für alle Domänen ausser der ersten Domäne der konstanten Region der schweren Kette von humanen Immunglobulinen, wie IgG, IgA, IgM bzw. IgE, kodiert.

6. DNA-Sequenzen gemäss Anspruch 5, wobei besagte humane Immunglobuline IgM bzw. solche der Klasse IgG sind.

7. DNA-Sequenzen gemäss Anspruch 6, wobei besagte humane Immunglobuline solche vom Typ Ig1 bzw. Ig3 sind.

8. Von DNA-Sequenzen gemäss einem der Ansprüche 4-7 kodierte rekombinante Proteine, wie allelische Varianten, oder Deletions-, Substitutions- oder Additionsanaloge davon.

9. Vektoren, die DNA-Sequenzen gemäss einem der Ansprüche 4-7 enthalten und zur Expression der von diesen DNA-Sequenzen kodierten Proteinen in prokaryotischen- wie eukaryotischen Wirtssystemen geeignet sind.

10. Prokaryotische- wie eukaryotische Wirtssysteme, die mit einem Vektor gemäss Anspruch 9 transformiert worden sind.

11. Wirtssysteme gemäss Anspruch 10, wobei diese Säuger- oder Insektenzellen sind.

12. Gegen eine Verbindung gemäss einem der Ansprüche 1-3 oder 8 gerichtete Antikörper.

13. Ein Verfahren zur Isolierung einer Verbindung gemäss einem der Ansprüche 1-3, **dadurch gekennzeichnet, dass** man im wesentlichen die folgenden Reinigungsschritte nacheinander ausführt: Herstellung eines Zellextraktes, Immunaffinitätschromatographie und/oder ein- oder mehrfache Ligandenaffinitätschromatographie, HPLC und präparative SDS-PAGE und falls gewünscht, die so isolierten Verbindungen chemisch oder enzymatisch spaltet und/oder in geeignete Salze überführt.

14. Ein Verfahren zur Herstellung einer Verbindung gemäss Anspruch 8, das **dadurch gekennzeichnet** ist, dass man ein wie in Anspruch 10 oder 11 beanspruchtes transformiertes Wirtssystem in einem geeigneten Medium kultiviert und aus dem Wirtssystem selbst oder dem Medium solche Verbindungen isoliert.

15. Pharmazeutische Präparate, **dadurch gekennzeichnet, dass** sie eine oder mehrere Verbindung(en) gemäss einem der Ansprüche 1-3 oder 8, gewünschtenfalls in Kombination mit weiteren pharmazeutisch wirksamen Subtanzen und/oder nicht-toxischen, inerten, therapeutisch verträglichen Trägermaterialien enthalten.

16. Pharmazeutische Präparate zur Behandlung von Krankheiten, bei denen TNF involviert ist, wobei solche Präparate **dadurch gekennzeichnet** sind, dass sie eine oder mehrere Verbindung(en) gemäss einem der Ansprüche 1-3 oder 8, gewünschtenfalls in Kombination mit weiteren pharmazeutisch wirksamen Subtanzen und/oder nicht-toxischen, inerten, therapeutisch verträglichen Trägermaterialien enthalten.

17. Verwendung einer Verbindung gemäss einem der Ansprüche 1-3 oder 8 zur Behandlung von Krankheiten.

18. Verwendung einer Verbindung gemäss einem der Ansprüche 1-3 oder 8 zur Behandlung von Krankheiten, bei denen TNF involviert ist.

19. Eine wie in einem der Ansprüche 1-3 oder 8 beanspruchte Verbindung wann immer sie nach einem wie in Anspruch 13 oder 14 beanspruchten Verfahren hergestellt worden ist.
